# EUROPÄISCHE PATENTSCHRIFT

(11) **EP 2 335 557 B1**
(45) Veröffentlichungstag und Bekanntmachung des Hinweises auf die Patenterteilung: **22.04.2015**
(21) Anmeldenummer: 10194653.1
(22) Anmeldetag: 13.12.2010
(51) Int. Cl.: A61B 1/04, G01N 21/27, A61B 5/00, A61B 5/1495, G01N 21/64, A61B 1/00

(54) **Verfahren zum Prüfen eines optischen Untersuchungssystems**
Method for checking an optical inspection system
Procédé de contrôle d'un système d'analyse optique

(30) Priorität: 16.12.2009 DE 102009058662
(43) Veröffentlichungstag der Anmeldung: 22.06.2011
(73) Patentinhaber: Karl Storz GmbH & Co. KG, 78532 Tuttlingen (DE)
(72) Erfinder: Beck, Gerd, 78532, Tuttlingen (DE); Ehrhardt, André, 78532, Tuttlingen (DE); Martin, Uwe, 78532, Tuttlingen (DE); Glöggler, Bernhard, 78532, Tuttlingen (DE); Schachenmayr, Hilmar, 78532, Tuttlingen (DE); Kennedy, Bryan, 78532, Tuttlingen (DE); Coussa, Raymond, 78532, Tuttlingen (DE); Tang, Nathan, 78532, Tuttlingen (DE)

(56) Entgegenhaltungen:
- EP-A1- 1 728 464
- DE-A1- 19 638 809
- US-A1- 2003 078 477
- US-A1- 2003 105 195
- US-A1- 2007 012 885

## Beschreibung

Die vorliegende Erfindung bezieht sich auf ein Verfahren zum Prüfen eines optischen Untersuchungssystems mit einer Lichtquelle, einer bildgebenden Einrichtung und einer Kamera zur optischen Untersuchung eines Objekts innerhalb und außerhalb der Medizin. Die vorliegende Erfindung bezieht sich insbesondere auf ein Verfahren zum Prüfen eines Endoskopiesystems mit einer Lichtquelle, einem Endoskop und einer Kamera.

Zur Endoskopie in medizinischen oder nicht-medizinischen Anwendungen - im zweiten Fall auch als Boroskopie bezeichnet - werden Endoskopiesysteme aus jeweils einem Endoskop und einer Lichtquelle verwendet. Die Lichtquelle kann in das Endoskop, insbesondere in dessen distales Ende, integriert sein oder als separate Einheit bereitstehen, die über ein Lichtleitkabel mit dem Endoskop optisch gekoppelt ist. Licht der Lichtquelle tritt am distalen Ende des Endoskops aus und beleuchtet dort ein zu betrachtendes Objekt. Vom Objekt remittiertes Licht wird von einer Optik am distalen Ende des Endoskops aufgefangen und auf einen lichtempfindlichen Bildsensor geleitet oder, beispielsweise mittels eines geordneten Bündels von Lichtwellenleitern oder einer Stablinsenoptik, zum proximalen Ende des Endoskops übertragen. Im zweiten Fall ist das vom Objekt remittierte Licht am proximalen Ende des Endoskops über ein Okular beobachtbar oder wird mittels einer Kamera erfasst. Alternativ oder zusätzlich zu remittiertem Licht kann auch vom Objekt emittiertes Licht beobachtet werden, insbesondere Fluoreszenzlicht.

Die Qualität eines mittels eines Endoskopiesystems erfassten Bilds, insbesondere Helligkeit, Helligkeits- und Farbkontrast, Signal-Rausch-Abstand, Farbtreue und Auflösung bzw. Schärfe, hängt vom betrachteten Objekt, insbesondere seinen optischen Eigenschaften, und vor allem vom Endoskopiesystem ab. Relevante Faktoren sind beispielsweise die Funktionsfähigkeit der Lichtquelle, ihre Strahlungsleistung bzw. der von ihr erzeugte Lichtstrom, das Spektrum des erzeugten Lichts, ggf. die Übertragungseigenschaften eines verwendeten Lichtleitkabels und der Kopplung des Lichtleitkabels mit der Lichtquelle und dem Endoskop, die Funktionsfähigkeit der Lichtübertragung innerhalb des Endoskops, der Wirkungsgrad der Auskopplung des Lichts der Lichtquelle aus dem Endoskop, die Funktionsfähigkeit bzw. die optischen Eigenschaften des Beobachtungs-Strahlengangs im Endoskop, ggf. einschließlich eines geordneten Bündels von Lichtwellenleitern oder einer Stablinsenoptik, die Funktionsfähigkeit des Okulars oder der Kamera. Häufige Fehlerquellen bilden u.a. die einem Alterungsprozess unterworfene Lichtquelle, ggf. das Lichtleitkabel und seine Kopplung an die Lichtquelle und das Endoskop und die Kopplung einer Kamera an das Endoskop.

Insbesondere für medizinisch-diagnostische Zwecke wird Fluoreszenzlicht beobachtet. In der Photodynamischen Diagnostik (PDD) wird beispielsweise eine durch verabreichtes 5-Aminolävulinsäure (ALA) induzierte Fluoreszenz von Protoporphyrin IX beobachtet. Die Anreicherung von ALA und damit auch die Intensität der Fluoreszenz sind vom Zustand des Gewebes abhängig. Bei der Autofluoreszenz-Diagnostik (AF-Diagnostik) wird die Fluoreszenz von körpereigenen Fluorophoren beobachtet, deren Konzentration ebenfalls vom Zustand des Gewebes abhängig ist. Auch außerhalb der Medizin werden fluoreszenzdiagnostische Verfahren verwendet.

Damit remittiertes Anregungslicht bzw. Beleuchtungslicht die Fluoreszenz nicht überstrahlt, werden im Beleuchtungs- bzw. Anregungs-Strahlengang zwischen Lichtquelle und Objekt ein Beleuchtungsfilter und im Beobachtungs-Strahlengang zwischen Objekt und Kamera bzw. Okular ein Beobachtungsfilter verwendet. Das Beleuchtungsfilter ist ein Kurzpassfilter, das im wesentlichen nur die zur Anregung der Fluoreszenz erforderlichen kurzen Wellenlängen transmittiert, längere Wellenlängen hingegen überwiegend oder fast ausschließlich reflektiert oder absorbiert. Eine sehr geringe, aber nicht verschwindende Transmission im Blockbereich ist bei manchen Anwendungen erwünscht, um auch ohne Fluoreszenz ein Bild zu erhalten, das eine geringe Helligkeit aufweist aber sichtbar ist. Das Beobachtungsfilter ist ein Langpassfilter, das nur Wellenlängen der Fluoreszenz transmittiert und vom Objekt remittiertes kurzwelliges Beleuchtungslicht reflektiert oder absorbiert. Beleuchtungsfilter bzw. Anregungsfilter können in der Regel manuell oder maschinell ausgetauscht bzw. gewechselt werden. Beobachtungsfilter können austauschbar bzw. wechselbar sein, sind aber in vielen Fällen fest in das Endoskop eingebaut. Beispielsweise in der Urologie werden für die Beobachtung in Weißlicht, ALA- oder AF-Fluoreszenz verschiedene Endoskope verwendet, die zumindest im Beobachtungs-Strahlengang für ihre jeweilige Verwendung optimiert sind bzw. eine entsprechende Filtercharakteristik aufweisen. Zu den oben genannten Fehlerquellen bzw. Einflüssen auf die Funktionsfähigkeit des Endoskopiesystems tritt im Fall der Beobachtung von Fluoreszenz somit noch die Kombination des Beleuchtungsfilters bzw. des Spektrums der Lichtquelle einerseits und des Beobachtungsfilters andererseits.

Eine entsprechende Problemstellung existiert bei anderen optischen Untersuchungssystemen, die eine bildgebende Einrichtung, eine Lichtquelle und eine Kamera zur optischen Untersuchung medizinischer und nicht-medizinischer Objekte in remittiertem Licht und/oder in Fluoreszenzlicht umfassen. Dazu zählen Exoskope, die beispielsweise zur Diagnostik und für mikrochirurgische Eingriffe an oder nahe Körperoberflächen verwendet werden.

In der DE 196 38 809 A1 ist eine Vorrichtung zur Prüfung und/oder Justierung eines PDD-oder PDT-Systems (PDT = Photodynamische Therapie) und/oder zur Schulung an einem derartigen System beschrieben. In einem Gehäuse ist ein Target angeordnet, dem gegenüber ein distales Ende eines Endoskops angeordnet werden kann. Die Krümmung des Targets kann der Objektfeldkrümmung einer bildgebenden Einheit des Endoskops entsprechen. Im Target sind ein Fotoelement und Lichtquellen vorgesehen. Das Fotoelement erfasst die Beleuchtungsstärke eines vom Endoskop ausgehenden Anregungslichts. Eine Steuerung steuert die Lichtquellen als Funktion der vom Fotoelement erfassten Beleuchtungsstärke.

In der DE 198 55 853 A1 ist eine Vorrichtung zur Prüfung und/oder Justierung eines PDD- oder PDT-Systems und/oder zur Schulung an einem derartigen System beschrieben. Die Vorrichtung umfasst ein Lumineszenzphantom mit einem Fluoreszenzfarbstoff. Gegenüber dem Lumineszenzphantom kann ein Ende eines Endoskops angeordnet werden.

In der nachveröffentlichten DE 10°2009°043°696 sind eine Vorrichtung und ein Verfahren zum Prüfen von Endoskopen beschrieben. Die Vorrichtung umfasst ein Filtermodul mit mehreren Durchbrüchen, in denen optische Filter angeordnet sind. Das Filtermodul wird aus einer Richtung durch die Lichtquelle über ein Lichtleitkabel beleuchtet. Aus einer entgegengesetzten Richtung wird das von dem Filtermodul transmittierte Licht mittels eines Endoskops beobachtet.

In US 2003/0105195 A1 sind eine Vorrichtung zum Kalibrieren eines optischen Scanners und ein Verfahren zu dessen Verwendung beschrieben. Im Mittelpunkt steht die Kalibrierung optischer Scanner für fluoreszente Proben und dabei die Kalibrierung der Empfindlichkeit eines optischen Detektors, des Abstands zwischen einem Objekttisch und einem Objektiv, der Geschwindigkeit der Bewegung des Objekttisches, des Scannerspiegels. Die Vorrichtung zum Kalibrieren umfasst ein Substrat mit einer Polymerschicht mit einem fluoreszenten Agens (ebenda). Die Oberfläche der Vorrichtung zum Kalibrieren wird mit Strahlung zur Anregung von Fluoreszenz des fluoreszenten Agens bestrahlt, wobei Fluoreszenzdaten erhalten werden, mittels derer der Scanner kalibriert wird.

Abhängig von konkreten, in der Praxis sich ergebenden Aufgabenstellungen kann jede der bislang bekannten Vorrichtungen und Verfahren Vor- und Nachteile aufweisen. Beispielsweise ermöglicht unter einigen Bedingungen und für einige Anwendungen keine der beschriebenen Vorrichtungen und Verfahren eine zuverlässige Prüfung eines vollständigen Endoskopiesystems oder eines vollständigen anderen optischen Untersuchungssystems in genau dem Zustand, in dem es davor oder danach medizinisch oder nicht-medizinisch verwendet wurde oder wird.

Ein Nachteil der in DE 196 38 809 A1 und in DE 198 55 853 A1 beschriebenen Vorrichtungen und Verfahren besteht darin, dass die Kamera eine fehlerhaft geringe Bildhelligkeit automatisch nachregelt, sodass diese vom Kamerasystem teilweise oder ganz kompensiert wird und erst bei sehr geringer Beleuchtung das Bild erkennbar schlecht wird. Entsprechendes gilt für das menschliche Auge. Insbesondere bei der Fluoreszenzdiagnostik besteht aber bereits vorher die Gefahr einer Fehldiagnose, insbesondere des Übersehens eines Tumors.

Eine Aufgabe der vorliegenden Erfindung besteht darin, ein verbessertes Verfahren zum Prüfen eines optischen Untersuchungssystems zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, im Rahmen eines Prüfverfahrens einen Betriebszustand einer Kamera eines optischen Untersuchungssystems zu erfassen bzw. abzufragen und anhand des erfassten Betriebszustands die Funktionsfähigkeit oder eine andere Eigenschaft des optischen Untersuchungssystems zu bestimmen. Bei vielen Kameras wird der Betriebszustand abhängig von der vorliegenden Belichtungssituation durch den Bildsensor selbst oder durch eine innerhalb oder außerhalb der Kamera angeordnete Kamerasteuerung (CCU = camera control unit) eingestellt. Zum Betriebszustand werden hier insbesondere die Belichtungszeit sowie der Verstärkungsfaktor oder andere Parameter einer analogen Signalverstärkung im Bildsensor gezählt. Durch geeignete Algorithmen werden Belichtungszeit und Verstärkungsfaktor beispielsweise so eingestellt, dass die mittlere Helligkeitswerte des erfassten (in Form eines analogen oder digitalen elektrischen Signals vorliegenden) Bilds oder die Helligkeitswerte eines priorisierten Bereichs (beispielsweise in der Bildmitte) des erfassten Bilds einen vorbestimmten Wert annehmen.

Bei einem lichtempfindlichen Bildsensor (beispielsweise CCD- oder CMOS-Sensor) ist in der Regel sowohl die Belichtungszeit als auch die analoge Verstärkung der elektrischen Signale vor ihrer Digitalisierung einstellbar. Die längste mögliche Belichtungszeit ist dabei durch die Bildwiederholfrequenz vorgegeben. Wenn das von der abbildenden Optik auf dem Bildsensor erzeugte Bild hell ist, werden eine kurze Belichtungszeit und eine geringe oder minimale Verstärkung gewählt. Bei abnehmender Helligkeit des Bilds wird beispielsweise zunächst bei unveränderter geringer Verstärkung die Belichtungszeit verlängert, um ein möglichst geringes Rauschen zu erzielen. Die längstmögliche Belichtungszeit beträgt beispielsweise bei einer Bildwiederholfrequenz von 50 Hz (50 Ganz- oder Halbbilder pro Sekunde) etwas weniger als 20 ms. Wenn die Belichtungszeit nicht weiter verlängert werden kann, wird die Verstärkung erhöht. Dabei nimmt mit abnehmender Helligkeit des Bilds auf dem Bildsensor das Rauschen zu bzw. der Signal-Rausch-Abstand im Signal des Bildsensors ab. Wenn die Verstärkung bei abnehmender Helligkeit des Bilds auf dem Bildsensor einen Maximalwert erreicht hat, können nur noch die Helligkeitswerte des digitalisierten Bilds numerisch skaliert werden. Dadurch wird bei weiter abnehmender Helligkeit die Bildqualität drastisch reduziert.

Beim beschriebenen Beispiel können im Betriebszustand der Kamera drei Stufen unterschieden werden.

Bei Stufe 1 nimmt die Verstärkung einen geringen, insbesondere einen minimalen, Wert an. Alternativ wird die Verstärkung auf einen anderen vorbestimmten Wert, beispielsweise einen mittleren Wert eingestellt. Innerhalb der Stufe 1 wird eine Variation der Helligkeit durch eine Variation der Belichtungszeit kompensiert. Stufe 1 wird auch als Autoshutter-Modus bezeichnet.

Bei Stufe 2 weist die Belichtungszeit den maximalen bei der gegebenen Bildwiederholfrequenz möglichen Wert auf. Innerhalb der Stufe 2 wird eine Variation der Helligkeit des Bilds auf dem Bildsensor durch eine Variation der Verstärkung kompensiert. Stufe 2 wird auch als AGC-Modus (AGC = auto gain control = automatische Steuerung der Verstärkung) bezeichnet.

Bei Stufe 3 liegen die maximale Belichtungszeit und die maximale Verstärkung vor. Innerhalb der Stufe 3 hat eine Variation der Helligkeit des Bilds auf dem Bildsensor eine Variation der Helligkeitswerte des digitalen Bilds zur Folge. Diese können nur noch durch eine numerische Skalierung verbessert werden. Dabei nimmt nicht nur der Signal-Rausch-Abstand ab sondern auch die Auflösung der Helligkeitswerte.

Für viele Anwendungen ist die Stufe 1 uneingeschränkt geeignet, während die Stufe 2 nur eingeschränkt oder bis zu einem bestimmten Schwellenwert der Verstärkung und die Stufe 3 nicht geeignet ist. Wenn die Stufe 1 vorliegt, ist das optische Untersuchungssystem uneingeschränkt funktionsfähig. Die Strahlungsleistung der Lichtquelle, ggf. die Qualität des Lichtleitkabels und seine Kopplung an die Lichtquelle und das Endoskop sowie der weitere Beleuchtungs-Strahlengang und der Beobachtungs-Strahlengang sind angesichts des gegebenen Reflexionsgrades des betrachteten Objekts und seines Abstand von der bildgebenden Einrichtung ausreichend.

Innerhalb der Stufe 2 nimmt das Rauschen im vom Bildsensor erfassten digitalisierten Bild zu bzw. der Signal-Rausch-Abstand ab. Die Qualität des erfassten Bilds kann jedoch trotzdem innerhalb der gesamten Stufe 2 oder bis zu einem Schwellenwert der Verstärkung für eine vorbestimmte Anwendung ausreichend sein. In der Stufe 3 ist die Qualität des vom Bildsensor erfassten Bilds für viele Anwendungen und insbesondere für medizinische Anwendungen nicht mehr ausreichend.

Diese Aussagen können getroffen werden, ohne die einzelnen Teile des optischen Untersuchungssystems, beispielsweise Lichtquelle, Lichtleitkabel, Endoskop, Kamera und deren jeweilige Kopplung, absolut zu messen. Insbesondere sind deshalb auch keine kalibrierten Messeinrichtungen erforderlich. Ferner kann das optische Untersuchungssystem in genau dem Zustand geprüft werden, in dem es vor oder nach der Prüfung zur optischen Untersuchung eines Objekts verwendet werden kann. Insbesondere müssen auch keine Lichtleitkabel oder andere Verbindungen zur optischen oder elektrischen Kopplung getrennt, geändert oder umgesetzt werden. Ferner ist keine Kalibrierung der Kamera erforderlich, da keine absolute Messung der Helligkeit des Bilds auf dem Bildsensor der Kamera notwendig ist, sondern bereits aus dem Betriebszustand der Kamera auf die Funktionsfähigkeit des optischen Untersuchungssystems geschlossen werden kann.

Neben der Belichtungszeit und der Verstärkung können zur Anpassung an eine Belichtungssituation abweichend von dem beschriebenen Beispiel weitere Parameter variiert werden. Beispielsweise können automatisch durch die CCU oder von außen gesteuert die Bildwiederholfrequenz und damit die maximale Belichtungszeit variiert werden und/oder Gruppen aus einer variierenden Anzahl von Bildpunkten zusammengefasst werden, um bei reduzierter räumlicher Auflösung eine Verbesserung des Signal-Rausch-Abstands zu erzielen. Beide Parameter können beispielsweise gleichzeitig mit der Verstärkung innerhalb der Stufe 2 oder in einer oder zwei weiteren Stufen zwischen Stufe 2 und Stufe 3 variiert werden.

Für eine genauere und quantitative Beschreibung des Betriebszustands der Kamera kann aus der Belichtungszeit T und der Verstärkung G ein Belichtungsparameter E berechnet werden, beispielsweise nach der Formel E=a·T^{b}·G^{c}. Die Konstanten a, b und c resultieren beispielsweise aus Geometrie, Empfindlichkeit und anderen Eigenschaften des Kamerasystems und einer Normierung des Belichtungsparameters E, so dass beispielsweise bei der maximalen Belichtungszeit und der minimalen Verstärkung der Belichtungsparameter E=1 beträgt. Die Konstanten b, c liegen beispielsweise jeweils zwischen 0,3 und 3, insbesondere zwischen 0,5 und 2. Im einfachsten Fall gilt b=c=1 und damit E=a·T·G.

Abhängig vom Aufbau und der Funktionsweise der Kamera können für verschiedene Bereiche des Bildsensors bzw. für verschiedene Bereiche des von der Kamera erfassten Bilds oder für verschiedene Farbkanäle gleiche oder verschiedene Betriebszustände vorliegen. Bei der Prüfung des optischen Untersuchungssystems können in diesem Fall einer oder mehrere Bereiche oder Farbkanäle ausgewählt oder die Betriebszustände der Kamera in verschiedenen Bereichen oder Farbkanälen miteinander logisch oder algebraisch verknüpft werden.

Statt den Betriebszustand der Kamera durch Abfragen der vorliegenden Parameter zu erfassen, kann der Betriebszustand auch durch ein Bestimmen des Rauschpegels oder des Signal-Rausch-Abstands im erfassten Bild erfasst werden. Der Rauschpegel ist beispielsweise innerhalb der oben beschriebenen Stufe 1 im Wesentlichen konstant und nimmt innerhalb der Stufen 2 und 3 mit abnehmender Helligkeit des Bilds auf dem Bildsensor zu. Über den Rauschpegel kann deshalb der Betriebszustand der Kamera auch dann erfasst werden, wenn die genannten Parameter nicht oder nur teilweise direkt abgefragt werden oder werden können.

Bei einem Verfahren zum Prüfen eines optischen Untersuchungssystems mit einer Lichtquelle, einer bildgebenden Einrichtung und einer Kamera zur optischen Untersuchung eines Objekts wird eine Referenzoberfläche mit vorbestimmten Eigenschaften mit Beleuchtungslicht einer Lichtquelle beleuchtet. Ein Bild der Referenzoberfläche wird mittels der bildgebenden Einrichtung und der Kamera erfasst. Ein Betriebszustand der Kamera, der während des Erfassens des Bilds vorliegt, wird erfasst. Die Funktionsfähigkeit oder eine andere Eigenschaft des Untersuchungssystems wird anhand des erfassten Betriebszustands bestimmt.

Das optische Untersuchungssystem ist insbesondere ein Endoskopiesystem für medizinische oder technische bzw. nicht-medizinische Anwendungen, wobei die bildgebende Einrichtung ein Endoskop ist. Endoskope für nicht-medizinische Anwendungen werden auch als Boroskope bezeichnet. Die Kamera kann in Form einer separaten und mit der bildgebenden Einrichtung optisch gekoppelten Einheit vorliegen. Alternativ ist die Kamera beispielsweise in die bildgebende Einrichtung integriert.

Die Referenzoberfläche und ihre optischen Eigenschaften sind insbesondere zeitlich unveränderlich bzw. stabil. Die Referenzoberfläche kann näherungsweise ein Lambert-Strahler sein bzw. einfallendes Licht näherungsweise ideal diffus reflektieren. Die Lichtquelle kann in die bildgebende Einrichtung integriert sein, beispielsweise in Form einer Leuchtdiode am distalen Ende eines Endoskops. Alternativ kann die Lichtquelle als separate Einrichtung ausgeführt sein, die beispielsweise über ein Lichtleitkabel mit der bildgebenden Einrichtung gekoppelt ist oder deren Licht auf andere Weise auf das zu untersuchende Objekt bzw. die Referenzoberfläche geleitet wird.

Das beschriebene Prüfverfahren ermöglicht eine einfache und schnelle Prüfung der Funktionsfähigkeit eines optischen Untersuchungssystems. Kalibrierte oder geeichte Messgeräte sind nicht erforderlich.

Das beschriebene Prüfverfahren ist in vielen Fällen auf ein optisches Untersuchungssystem in dem Zustand anwendbar, in dem es davor oder danach, beispielsweise in der medizinischen Diagnostik zum Einsatz kommt. Mit dem beschriebenen Prüfverfahren kann deshalb auch beispielsweise auf einfache und schnelle Weise festgestellt werden, ob alle Komponenten des Untersuchungssystems funktionsfähig und einwandfrei miteinander gekoppelt sind. Beispielsweise hat eine unzureichende optische Kopplung einer externen, als separate Einrichtung ausgeführten Lichtquelle mit einem Endoskop aufgrund eines defekten Lichtleitkabels oder einer fehlerhaften Steckverbindung eine Auswirkung auf die Beleuchtung des zu untersuchenden Objekts bzw. der Referenzoberfläche. Die mangelhafte Beleuchtung wirkt sich auf den Betriebszustand der Kamera aus und kann deshalb mit dem beschriebenen Prüfverfahren erkannt werden.

Bei dem hier beschriebenen Verfahren wird die Referenzoberfläche nacheinander mit Beleuchtungslicht mit einem ersten Beleuchtungsspektrum und mit Beleuchtungslicht mit einem zweiten Beleuchtungsspektrum beleuchtet. Es werden ein erster Betriebszustand der Kamera, der während des Erfassens eines Bilds bei Beleuchten der Referenzoberfläche mit dem Beleuchtungslicht mit dem ersten Beleuchtungsspektrum vorliegt, und ein zweiter Betriebszustand der Kamera, der während des Erfassens eines Bilds bei Beleuchten der Referenzoberfläche mit dem Beleuchtungslicht mit dem zweiten Beleuchtungsspektrum vorliegt, erfasst. Die Funktionsfähigkeit oder eine andere Eigenschaft des optischen Untersuchungssystems wird anhand des ersten Betriebszustands und des zweiten Betriebszustands bestimmt. Dies kann besonders nützlich sein, wenn das optische Untersuchungssystem zur Erfassung von Fluoreszenz (beispielsweise in der PDD oder in der AF-Diagnostik) verwendet werden soll.

Beispielsweise ist die Lichtquelle durch manuell oder maschinell in den Beleuchtungs-Strahlengang einführbare Filter dazu ausgebildet, alternativ ein, zumindest näherungsweise, weißes Spektrum sichtbaren Lichts und eines oder mehrere Spektren zur Anregung von Fluoreszenz verschiedener Fluorophore bereitzustellen. Wenn die für das optische Untersuchungssystem vorgesehene Verwendung die Erfassung von Fluoreszenzlicht vorsieht, muss die bildgebende Einrichtung ein Beobachtungsfilter bzw. eine entsprechende Filtercharakteristik aufweisen, die an das Spektrum des Beleuchtungslichts bzw. an das Beleuchtungsfilter angepasst ist. Durch Beleuchten der Referenzoberfläche mit Beleuchtungslicht mit verschiedenen Spektren und Erfassen der dabei jeweils vorliegenden Betriebszustände der Kamera kann ermittelt werden, welches Beobachtungsfilter bzw. welche Filtercharakteristik der Beobachtungs-Strahlengang der bildgebenden Einrichtung aufweist. Dies ermöglicht eine einfache, schnelle und zuverlässige Verifizierung, ob das optische Untersuchungssystem das richtige Beobachtungsfilter oder die richtige bildgebende Einrichtung mit der richtigen Filtercharakteristik des Beobachtungs-Strahlengangs umfasst.

Bei einem Verfahren, wie es hier beschrieben ist, kann das distale Ende der bildgebenden Einrichtung an einer vorbestimmten Position, insbesondere auch in einer vorbestimmten Richtung, relativ zu der Referenzoberfläche angeordnet werden und während des Beleuchtens und des Erfassens des Bilds angeordnet sein. Diese Anordnung an einer vorbestimmten Position und ggf. in einer vorbestimmten Richtung kann durch eine Positionierungseinrichtung unterstützt werden, welche die bildgebende Einrichtung, insbesondere deren distales Ende, führt und hält. Alternativ sind beispielsweise auf der Referenzoberfläche eine oder mehrere optisch erkennbare Marken angeordnet, wobei das distale Ende der bildgebenden Einrichtung relativ zu der Referenzoberfläche so angeordnet wird, dass die Marken an vorbestimmten Orten im erfassten Bild, beispielsweise am Rand, liegen. Diese Marken können gleichzeitig weitere Funktionen übernehmen, beispielsweise die Fokussierung der bildgebenden Einrichtung auf die Referenzoberfläche oder - insbesondere aufgrund ihrer spektralen Eigenschaften - die Identifizierung von Beleuchtungs- und Beobachtungsfiltern des optischen Untersuchungssystems vereinfachen.

Die Anordnung des distalen Endes der bildgebenden Einrichtung an einer vorbestimmten Position und ggf. in einer vorbestimmten Richtung verbessert die Genauigkeit, mit der das optische Untersuchungssystem geprüft werden kann. Dazu entsprechen die vorbestimmte Position und ggf. die vorbestimmte Richtung beispielsweise dem typischen oder einem maximalen Abstand zwischen dem distalen Ende der bildgebenden Einrichtung und dem betrachteten Objekt bei einer für das optische Untersuchungssystem vorgesehenen Verwendung.

Bei jedem der hier beschriebenen Verfahren können ferner eine für das optische Untersuchungssystem vorgesehene Anwendung erfasst und eine Bedingung, die der vorgesehenen Anwendung des optischen Untersuchungssystems zugeordnet ist, ermittelt werden. Jeder Anwendung ist eine Bedingung so zugeordnet, dass die Funktionsfähigkeit des optischen Untersuchungssystems für die Anwendung oder eine andere vorbestimmte Eigenschaft des optischen Untersuchungssystems vorliegt, wenn der während des Erfassens des Bilds der Referenzoberfläche vorliegende Betriebszustand der zugeordneten Bedingung entspricht. Beispielsweise können medizinisch-diagnostische Untersuchungen in Hohlräumen unterschiedlicher Größe oder von Organen mit unterschiedlichen optischen Eigenschaften unterschiedliche Anforderungen an das optische Untersuchungssystem stellen. Diese unterschiedlichen Anforderungen können durch unterschiedliche Bedingungen an den während des Erfassens des Bilds der Referenzoberfläche vorliegenden Betriebszustand der Kamera zum Ausdruck kommen.

Bei jedem der hier beschriebenen Verfahren kann die Referenzoberfläche mit einer Bestrahlungsstärke bzw. Beleuchtungsstärke beleuchtet werden, die einen vorbestimmten Bruchteil der bei der vorgesehenen Verwendung des optischen Untersuchungssystems angewandten Bestrahlungsstärke beträgt. Dies wird beispielsweise durch Dimmen der Lichtquelle, durch Verwendung einer Blende, eines Gitters oder eines Filters erreicht. Durch diese reduzierte Bestrahlungsstärke wird beispielsweise dem Umstand Rechnung getragen, dass das bei der vorgesehenen Verwendung des optischen Untersuchungssystems betrachtete Objekt einen geringeren Remissionsgrad aufweist als die Referenzoberfläche. Zur Durchführung des Prüfverfahrens kann somit nicht nur die Referenzoberfläche an die vorgesehene Verwendung des zu prüfenden optischen Untersuchungssystems sondern alternativ die Bestrahlungsstärke der Referenzoberfläche an die vorgesehene Verwendung angepasst werden, wobei der zweite Weg abhängig von der Lichtquelle und deren Steuerung einfacher sein kann. Die hier beschriebenen Verfahren ermöglichen auf diese Weise eine sehr differenzierte Prüfung eines optischen Untersuchungssystems.

Bei jedem der hier beschriebenen Verfahren kann das Erfassen des Betriebszustands ein Ermitteln eines Belichtungsparameters und das Bestimmen der Funktionsfähigkeit oder einer anderen Eigenschaft ein Vergleichen des ermittelten Belichtungsparameters mit einem vorbestimmten Schwellenwert umfassen. Der Belichtungsparameter E wird beispielsweise nach der bereits beschriebenen Formel E=a·T^{b}·G^{c} aus der Belichtungszeit T und der Verstärkung G berechnet. Gegenüber der ebenfalls bereits beschriebenen Unterscheidung des Betriebszustands nach Stufen ermöglicht die Berechnung eines Belichtungsparameters eine genauere Quantifizierung und feinere Differenzierung. Der Schwellenwert ist beispielsweise von der vorgesehenen Verwendung des optischen Untersuchungssystems abhängig. Durch Wahl des Schwellenwerts können beispielsweise die optischen Eigenschaften eines bei der vorgesehenen Verwendung des optischen Untersuchungssystems zu betrachteten Objekts, insbesondere der Remissionsgrad, die Fluoreszenz-Quantenausbeute und deren Wellenlängenabhängigkeit, sowie Kontraste, und deren Unterschiede zur Referenzoberfläche berücksichtigt werden.

Bei jedem der hier beschriebenen Verfahren kann ferner ein erster Betriebszustand der Kamera während des Erfassens eines Bilds der Referenzoberfläche bei Verwendung eines ersten Beobachtungsfilters oder einer bildgebenden Einrichtung mit einer ersten Filtercharakteristik des Beobachtungs-Strahlengangs und ein zweiter Betriebszustand der Kamera während des Erfassens eines zweiten Bilds bei Verwendung eines zweiten Beobachtungsfilters bzw. einer bildgebenden Einrichtung mit einer zweiten Filtercharakteristik des Beobachtungs-Strahlengangs erfasst werden. Anhand der beiden Betriebszustände kann das Beleuchtungsspektrum bzw. das verwendete Beleuchtungsfilter bestimmt werden. Damit ist beispielsweise die einwandfreie Funktion einer maschinellen Steuerung der Beleuchtungsfilter verifizierbar oder ein manuell eingesetztes Beleuchtungsfilter identifizierbar.

Durch Erfassen mehrerer Betriebszustände der Kamera bei Erfassen von Bildern unter Verwendung verschiedener Beleuchtungsfilter und/oder Beobachtungsfilter können somit weitere detaillierte Informationen über Eigenschaften des optischen Untersuchungssystems gewonnen werden. Soweit das Beleuchtungsfilter bzw. das Beobachtungsfilter kontrolliert und reproduzierbar gewechselt werden kann, können somit eindeutige Rückschlüsse auf muss die bildgebende Einrichtung ein Beobachtungsfilter bzw. eine entsprechende Filtercharakteristik aufweisen, die an das Spektrum des Beleuchtungslichts bzw. an das Beleuchtungsfilter angepasst ist. Durch Beleuchten der Referenzoberfläche mit Beleuchtungslicht mit verschiedenen Spektren und Erfassen der dabei jeweils vorliegenden Betriebszustände der Kamera kann ermittelt werden, welches Beobachtungsfilter bzw. welche Filtercharakteristik der Beobachtungs-Strahlengang der bildgebenden Einrichtung auf weist. Dies ermöglicht eine einfache, schnelle und zuverlässige Verifizierung, ob das optische Untersuchungssystem das richtige Beobachtungsfilter oder die richtige bildgebende Einrichtung mit der richtigen Filtercharakteristik des Beobachtungs-Strahlengangs umfasst.

Bei jedem der hier beschriebenen Verfahren kann ferner ein erster Betriebszustand der Kamera während des Erfassens eines Bilds der Referenzoberfläche bei Verwendung eines ersten Beobachtungsfilters oder einer bildgebenden Einrichtung mit einer ersten Filtercharakteristik des Beobachtungs-Strahlengangs und ein zweiter Betriebszustand der Kamera während des Erfassens eines zweiten Bilds bei Verwendung eines zweiten Beobachtungsfilters bzw. einer bildgebenden Einrichtung mit einer zweiten Filtercharakteristik des Beobachtungs-Strahlengangs erfasst werden. Anhand der beiden Betriebszustände kann das Beleuchtungsspektrum bzw. das verwendete Beleuchtungsfilter bestimmt werden. Damit ist beispielsweise die einwandfreie Funktion einer maschinellen Steuerung der Beleuchtungsfilter verifizierbar oder ein manuell eingesetztes Beleuchtungsfilter identifizierbar.

Durch Erfassen mehrerer Betriebszustände der Kamera bei Erfassen von Bildern unter Verwendung verschiedener Beleuchtungsfilter und/oder Beobachtungsfilter können somit weitere detaillierte Informationen über Eigenschaften des optischen Untersuchungssystems gewonnen werden. Soweit das Beleuchtungsfilter bzw. das Beobachtungsfilter kontrolliert und reproduzierbar gewechselt werden kann, können somit eindeutige Rückschlüsse auf das jeweils andere Filter und/oder auf die Funktionsfähigkeit einer Einrichtung zum Wechseln des Filters gezogen werden.

Bei jedem der hier beschriebenen Verfahren, bei dem mehrere Betriebszustände bei verschiedenen Beleuchtungsfiltern und/oder bei verschiedenen Beobachtungsfiltern erfasst werden, kann das Bestimmen der Funktionsfähigkeit oder der anderen Eigenschaft des optischen Untersuchungssystems ein Ermitteln, insbesondere ein Berechnen, eines Indikatorparameters aus den erfassten Betriebszuständen und ein Vergleichen des ermittelten Indikatorparameters mit einem, insbesondere mehreren, Schwellenwerten umfassen. Beispielsweise wird aus jedem der erfassten Betriebszustände, wie oben beschrieben, ein Belichtungsparameter ermittelt. Die Belichtungsparameter werden dann logisch oder algebraisch verknüpft, beispielsweise durch Bilden eines Quotienten oder einer Differenz, welche den Indikatorparameter bildet.

Bei jedem der hier beschriebenen Verfahren kann das Erfassen eines Betriebszustands das Erfassen von je einem Betriebszustand für jeden von einer Mehrzahl von Farbkanälen umfassen. Ferner kann das Erfassen eines Betriebszustands das Ermitteln je eines Belichtungsparameters für jeden der Mehrzahl von Farbkanälen umfassen. Dies gilt insbesondere dann, wenn die verwendete Kamera die unabhängige Einstellung verschiedener Belichtungszeiten und/oder Verstärkungen für verschiedene Farbkanäle vorsieht oder ermöglicht. Die den verschiedenen Farbkanälen zugeordneten erfassten Betriebszustände bzw. Belichtungsparameter können wiederum logisch oder algebraisch verknüpft und dann beispielsweise mit einem Schwellenwerten verglichen werden, um die Funktionsfähigkeit oder eine andere Eigenschaft des optischen Untersuchungssystems zu bestimmen. Insbesondere werden die erfassen Betriebszustände bzw. Belichtungsparameter logisch oder algebraisch verknüpft und dann mit mehreren Schwellenwerten verglichen.

Bei jedem der hier beschriebenen Verfahren kann das Erfassen eines Betriebszustands ein Erfassen eines Weißabgleich-Parameters umfassen. Weißabgleich-Parameter können bei einem vorangehenden manuellen oder - automatisch oder manuell ausgelösten oder gesteuerten - automatischen Weißabgleich gewonnen bzw. eingestellt worden sein. Weißabgleich-Parameter beschreiben bzw. bestimmen beispielsweise die Verhältnisse der Belichtungszeiten, Verstärkungen oder Belichtungsparameter der einzelnen Farbkanäle oder stellen bei oder nach der Digitalisierung des Bilds anzuwendende Korrekturfaktoren dar. Besonders leicht und zuverlässig ist anhand der Weißabgleich-Parameter beispielsweise bestimmbar, ob das Beleuchtungslicht der Lichtquelle (näherungsweise) weiß ist oder zur Anregung von Fluoreszenz überwiegend kurze Wellenlängen umfasst.

Abhängig von den optischen Eigenschaften der Referenzoberfläche können ferner auf der Grundlage der den einzelnen Farbkanälen zugeordneten Betriebszustände oder Belichtungsparameter oder auf der Grundlage von einem oder mehreren Weißabgleich-Parametern detaillierte Informationen über die Eigenschaften des Untersuchungssystems, insbesondere Beleuchtungsfilter und Beobachtungsfilter, gewonnen werden.

Die Referenzoberfläche kann bei jedem der hier beschriebenen Verfahren weiß oder grau sein bzw. einen, insbesondere innerhalb des für das menschliche Auge sichtbaren Spektralbereichs, im Wesentlichen wellenlängenunabhängigen Remissionsgrad aufweisen. Alternativ kann die Referenzoberfläche farbig sein bzw. einen wellenlängenabhängigen Remissionsgrad aufweisen. Alternativ oder zusätzlich kann die Referenzoberfläche fluoreszierend sein. Die Referenzoberfläche kann einheitliche bzw. homogene optische Eigenschaften oder mehrere Bereiche mit unterschiedlichen optischen Eigenschaften aufweisen. Wie unten anhand der Figuren näher beschrieben ist, kann eine Referenzoberfläche mit mehreren Bereichen mit unterschiedlichen optischen Eigenschaften eine noch detailliertere oder genauere Bestimmung von Eigenschaften des optischen Untersuchungssystems ermöglichen.

Bei jedem der hier beschriebenen Verfahren kann das Erfassen eines Betriebszustands ein Erfassen eines Rauschpegels oder eines Signal-Rausch-Abstands in dem erfassten Bild umfassen. Aus dem Rauschpegel kann, wie bereits beschrieben, auf den Betriebszustand der Kamera geschlossen werden. Der Betriebszustand der Kamera kann damit auch erfasst werden, wenn der oder die ihn bestimmenden Parameter nicht direkt abgefragt werden können oder auf andere Weise zugänglich sind.

Bei jedem der hier beschriebenen Verfahren können ferner Patientendaten erfasst und Information über die Funktionsfähigkeit oder eine andere Eigenschaft des optischen Untersuchungssystems und die Patientendaten in einer Datenbank abgelegt werden. Durch Integration des Prüfverfahrens mit dem Erfassen und Speichern der Patientendaten kann sichergestellt werden, dass bei jeder medizinisch-diagnostischen Verwendung des optischen Untersuchungssystems dieses auch hinsichtlich seiner Funktionsfähigkeit und/oder einer anderen Eigenschaft geprüft und das Ergebnis der Prüfung individuell bzw. auf den Patienten bezogen dokumentiert wird. Das Prüfverfahren kann damit zu einem zuverlässigen und nicht manipulierbaren Bestandteil der Qualitätssicherung im klinischen Alltag werden.

Bei jedem der hier beschriebenen Prüfverfahren kann nach dem Bestimmen der Funktionsfähigkeit oder einer anderen Eigenschaft des optischen Untersuchungssystems eine Meldung angezeigt werden. Diese Meldung nennt die Funktionsfähigkeit oder den Grad der Funktionsfähigkeit und/oder einer anderen bestimmten Eigenschaft des Untersuchungssystems. Alternativ oder zusätzlich kann die Meldung eine Handlungsanweisung oder eine Handlungsempfehlung enthalten. Beispielsweise enthält die Meldung eine Aufforderung, Steckverbindungen zwischen einem Lichtleitkabel und der Lichtquelle bzw. der bildgebenden Einrichtung zu prüfen oder die bildgebende Einrichtung oder ein Beobachtungsfilter auszutauschen und die Prüfung des optischen Untersuchungssystems anschließend zu wiederholen. Nach wiederholter Feststellung mangelnder Funktionsfähigkeit können weitere Anweisungen oder Empfehlungen gemeldet werden, beispielsweise eine Empfehlung zum sofortigen oder baldigen Austausch der Lichtquelle.

Die vorliegende Erfindung ist als Verfahren oder als Computer-Programm mit Programmcode zur Durchführung oder Steuerung eines solchen Verfahrens, wenn das Computer-Programm auf einem Computer oder einem Prozessor abläuft, implementierbar. Ferner ist die Erfindung als Computer-Programm-Produkt mit auf einem maschinenlesbaren Träger (beispielsweise einem ROM-, PROM-, EPROM-, EEPROM- oder Flash-Speicher, einer CD-ROM, DVD, HD-DVD, Blue-Ray-DVD, Diskette oder Festplatte) oder in Form von Firmware gespeichertem Programmcode zur Durchführung von einem der genannten Verfahren, wenn das Computer-Programm-Produkt auf einem Computer, Rechner oder Prozessor abläuft, implementierbar. Ferner kann die vorliegende Erfindung als digitales Speichermedium (beispielsweise ROM-, PROM-, EPROM-, EEPROM- oder Flash-Speicher, CD-ROM, DVD, HD-DVD, Blue-Ray-DVD, Diskette oder Festplatte) mit elektronisch auslesbaren Steuersignalen, die so mit einem programmierbaren Computer- oder Prozessor-System zusammenwirken können, dass eines der beschriebenen Verfahren ausgeführt wird, implementiert werden.

Ferner kann die vorliegende Erfindung als Steuerung für ein optisches Untersuchungssystem mit einer bildgebenden Einrichtung, insbesondere einem Endoskop, einer Kamera und einer Lichtquelle zur optischen Untersuchung eines Objekts implementiert werden, wobei die Steuerung ausgebildet ist, um eines der beschriebenen Verfahren auszuführen, oder wobei die Steuerung ein Computer-Programm, ein Computer-Programm-Produkt oder ein digitales Speichermedium umfasst, wie sie im vorangehenden Absatz beschrieben wurden. Die Steuerung kann eine Kamerasteuerung oder in diese integriert sein.

### Kurzbeschreibung der Figuren

Nachfolgend werden Ausführungsformen anhand der beigefügten Figuren näher erläutert. Es zeigen:
- Figur 1: eine schematische Darstellung eines optischen Untersuchungssystems;
- Figur 2: eine schematische Darstellung eines Endoskops mit einer Prüfvorrichtung;
- Figur 3: eine schematische Darstellung mehrerer Spektren;
- Figur 4: eine schematische Darstellung weiterer Spektren;
- Figur 5: eine schematische Darstellung von Produkten von Transmissionsspektren;
- Figur 6: eine schematische Darstellung von Weißabgleich-Parametern;
- Figur 7: eine schematische Darstellung weiterer Weißabgleich-Parameter;
- Figur 8: eine schematische Darstellung eines Flussdiagramms.

### Beschreibung der Ausführungsformen

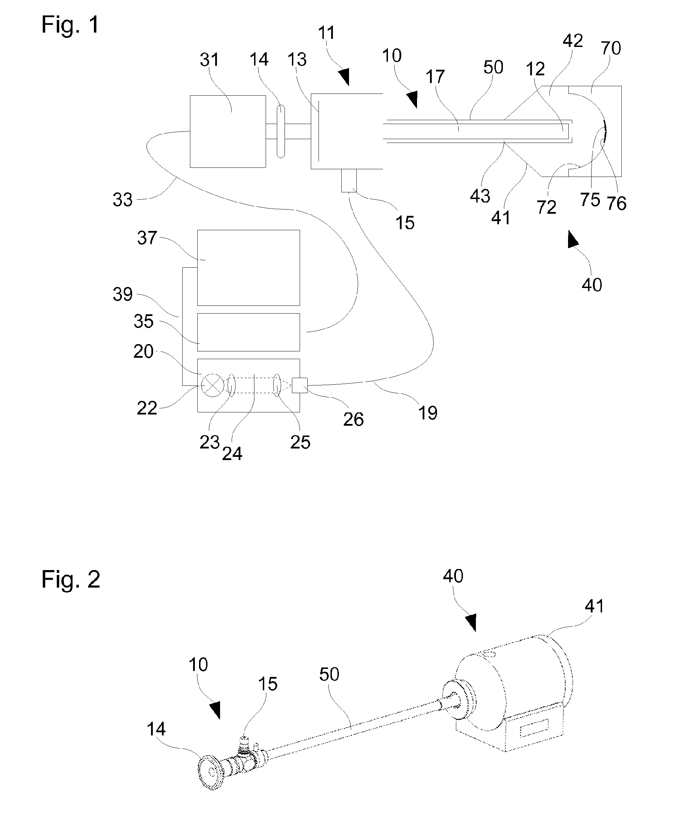

Figur 1 zeigt eine schematische Darstellung eines optischen Untersuchungssystems. Das optische Untersuchungssystem ist in diesem Beispiel ein Endoskopiesystem, das beispielsweise bei medizinisch-diagnostischen Verfahren in der Urologie und in anderen Fachgebieten einsetzbar ist. Das Endoskopiesystem umfasst ein Endoskop 10 mit einem proximalen Ende 11 und einem distalen Ende 12. Das Endoskop 10 umfasst einen Beleuchtungs- bzw. Anregungs-Strahlengang und einen Beobachtungs-Strahlengang, die in Figur 1 nicht im Detail dargestellt sind. Der Beleuchtungs-Strahlengang umfasst insbesondere einen oder mehrere Lichtwellenleiter zum Übertragen von Beleuchtungs- bzw. Anregungslicht vom proximalen Ende 11 zum distalen Ende 12 und einen Lichtaustritt am distalen Ende 12, durch den Beleuchtungslicht aus dem distalen Ende 12 des Endoskops 10 austreten kann, um ein zu betrachtendes Objekt zu beleuchten. Der Beobachtungs-Strahlengang umfasst einen Lichteintritt am distalen Ende 12 des Endoskops 10, eine Optik zum Übertragen von Beobachtungslicht, das von einem betrachteten Objekt ausgeht, vom distalen Ende 12 zum proximalen Ende 11, ein Beobachtungsfilter 13 und ein Okular 14. Zum Übertragen des Beobachtungslichts vom distalen Ende 12 zum proximalen Ende 11 des Endoskops 10 ist beispielsweise eine Stablinsenoptik oder ein geordnetes Bündel von Lichtwellenleitern in einem Schaft 17 des Endoskops 10 vorgesehen. Das Endoskop 10 weist ferner am proximalen Ende 11 eine Kupplung 15 zum mechanischen und optischen Koppeln eines Lichtleitkabels 19 mit dem beschriebenen Beleuchtungs-Strahlengang im Endoskop 10 auf.

Das Endoskop 10 ist über das Lichtleitkabel 19 mit einer Lichtquellenvorrichtung 20 gekoppelt. Die Lichtquellenvorrichtung 20 umfasst eine Lichtquelle 22, beispielsweise eine Halogen-Glühlampe, eine Hochdruck-Gasentladungslampe, eine Leuchtdiode oder einen Laser. Ferner umfasst die Lichtquellenvorrichtung 20 eine erste Sammellinse 23, ein Beleuchtungsfilter 24 und eine zweite Sammellinse 25. Die Lichtquelle 22 ist über die erste Sammellinse 23, das Beleuchtungsfilter 24, die zweite Sammellinse 25 und eine Kupplung 26 mit dem Lichtleitkabel 19 gekoppelt.

Eine Kamera 31 ist über das Okular 14 mit dem Endoskop 10 und dessen Beobachtungs-Strahlengang mechanisch bzw. optisch gekoppelt. Die Kamera 31 umfasst einen lichtempfindlichen Bildsensor, beispielsweise einen CCD- oder CMOS-Sensor, zum Wandeln von auf den Bildsensor fallendem Licht in analoge oder digitale elektrische Signale. Die Kamera 31 ist über ein Signalkabel 33 zur Übertragung analoger oder digitaler elektrischer oder optischer Signale mit einer Kamerasteuerung 35 gekoppelt, die auch als CCU = camera control unit bezeichnet wird.

Die Lichtquellenvorrichtung 20, die Kamerasteuerung 35 und ein Bildschirm 37 sind über einen Kommunikationsbus 39 oder mehrere separate Signalleitungen miteinander gekoppelt. Über den Kommunikationsbus 39 können weitere, in Figur 1 nicht dargestellte Vorrichtungen innerhalb oder außerhalb des Behandlungsraums, in dem das Endoskopiesystem angeordnet ist, mit der Lichtquellenvorrichtung 20, der Kamerasteuerung 35 und dem Bildschirm 37 gekoppelt sein, beispielsweise eine Datenbank, eine Tastatur, eine Computermaus und andere Benutzerschnittstellen.

In Figur 1 ist ferner eine Prüfvorrichtung 40 mit einem lichtdichten Gehäuse 41, einem Hohlraum 42 in dem lichtdichten Gehäuse 41 und einer Öffnung 43 zum Hohlraum 42 dargestellt. Das distale Ende 12 des Endoskops 10 ist durch die Öffnung 43 in den Hohlraum 42 der Prüfvorrichtung 40 eingeführt. Eine in der Öffnung 43 angeordnete Positionierungseinrichtung 50 hält form- und/oder kraftschlüssig den Schaft 17 des Endoskops 10 so, dass das distale Ende 12 des Endoskops 10 an einer vorbestimmten Position und in einer vorbestimmten Richtung im Hohlraum 42 angeordnet ist. Ferner verhindert die Positionierungseinrichtung 50, zumindest wenn der Schaft 17 des Endoskops 10 in der Positionierungseinrichtung 50 angeordnet ist, weitgehend das Eindringen von Licht aus der Umgebung durch die Öffnung 43 in den Hohlraum 42 im Gehäuse 41.

Im Hohlraum 42 der Prüfvorrichtung 40 ist ferner ein Referenzkörper 70 mit einer Referenzoberfläche 72 angeordnet. Die Referenzoberfläche 72 weist vorbestimmte optische Eigenschaften und die räumliche Gestalt eines Ausschnitts aus einer Kugeloberfläche oder aus einem Kreiszylindermantel auf. Die für das distale Ende 12 des Endoskops 10 vorgesehene Position liegt insbesondere am Mittelpunkt dieser Kugeloberfläche bzw. an der Symmetrieachse des Kreiszylindermantels. Insbesondere liegt der objektseitige Hauptpunkt bzw. der Schnittpunkt der optischen Achse mit der objektseitigen Hauptebene der bildgebenden Einrichtung 10 am Mittelpunkt der Kugeloberfläche bzw. an der Symmetrieachse des Kreiszylindermantels.

Die Referenzoberfläche 72 weist zeitlich unveränderliche bzw. stabile vorbestimmte optische Eigenschaften auf. Die Referenzoberfläche 72 kann weiß sein bzw. einen Remissionsgrad aufweisen, der im für das menschliche Auge sichtbaren Spektralbereich im Wesentlichen wellenlängenunabhängig ist. Die Referenzoberfläche 72 kann alternativ farbig sein bzw. einen im für das menschliche Auge sichtbaren Spektralbereich wellenlängenabhängigen Remissionsgrad aufweisen. Alternativ oder zusätzlich kann die Referenzoberfläche 72 fluoreszierend sein. Dabei liegen die zur Anregung der Fluoreszenz erforderlichen Wellenlängen beispielsweise im ultravioletten oder, für medizinische Anwendungen bevorzugt, im blauen Spektralbereich und das emittierte Fluoreszenzlicht im grünen, roten oder infraroten Spektralbereich. Die optischen Eigenschaften können über die gesamte Referenzoberfläche 72 homogen bzw. ortsunabhängig sein.

Alternativ weist die Referenzoberfläche mehrere Bereiche mit verschiedenen optischen Eigenschaften auf. Bei dem in Figur 1 dargestellten Beispiel ist die Referenzoberfläche 72 überwiegend weiß mit einem Indikatorbereich 75 und einem Referenzbereich 76, die jeweils vom Rest der Referenzoberfläche 72 verschiedene optische Eigenschaften aufweisen. Der Indikatorbereich 75 und der Referenzbereich 76 können mit scharfen Rändern oder aufgrund ihrer Anordnung oder Gestalt eine Fokussierung oder eine Einstellung der Brennweite bzw. der Größe des Sichtfelds der bildgebenden Einrichtung vereinfachen oder ermöglichen. Darüber hinaus können die optischen Eigenschaften des Indikatorbereichs 75 und des Referenzbereichs 76 eine Bestimmung des Transmissionsspektrums des Beleuchtungsfilters 24 und des Transmissionsspektrums des Beobachtungsfilters 13 vereinfachen.

Der Referenzkörper 70 besteht, von dem Indikatorbereich und dem Referenzbereich 75 an der Referenzoberfläche 72 abgesehen, insbesondere aus Polytetrafluorethylen PTFE, das beispielsweise unter dem Markennamen Teflon von DuPont vertrieben wird, oder aus Silikon. Sowohl PTFE als auch Silikon kann mit weißen oder farbigen Pigmenten oder Farbstoffen gefüllt sein.

Figur 2 zeigt eine schematische axonometrische Darstellung eines Endoskops 10 und einer Prüfvorrichtung 40, die dem Endoskop und der Prüfvorrichtung ähnlich sind, die oben anhand der Figur 1 dargestellt wurden. Im Unterschied zur Figur 1 sind keine separate Lichtquelle, Kamera oder andere Vorrichtungen gezeigt. Die exakte Positionierung des distalen Endes des Endoskops 10 in der Prüfvorrichtung 40 wird bei diesem Beispiel durch Formschluss zwischen der Positionierungseinrichtung 50 und dem distalen Ende 11 des Endoskops 10 erreicht, insbesondere mittels eines mechanischen Anschlags und/oder einer Rastverbindung.

Die nachfolgend beschriebenen Prüfverfahren sind auch auf optische Untersuchungssysteme und Prüfvorrichtungen anwendbar, die sich von den oben anhand der Figuren 1 und 2 dargestellten unterscheiden. Beispielsweise sind die Prüfverfahren unabhängig davon anwendbar, ob eine Lichtquelle und/oder eine Kamera als separate und mit dem Endoskop koppelbare Einheiten ausgeführt oder in das Endoskop an dessen proximalem oder distalem Ende integriert sind. Ferner sind die Prüfverfahren anwendbar, wenn das Anregungs- bzw. Beleuchtungslicht nicht über das Endoskop bzw. allgemeiner über die bildgebende Einrichtung, sondern auf andere Weise auf das zu betrachtende Objekt bzw. die Referenzoberfläche geleitet wird. Auch die Anordnung von Beleuchtungs- und Beobachtungsfiltern kann von den oben anhand der Figuren 1 und 2 dargestellten Beispielen abweichen. Um das Verständnis zu vereinfachen, werden nachfolgend dennoch beispielhaft Bezugszeichen aus den Figuren 1 und 2 verwendet.

Bei einem ersten Prüfverfahren für ein optisches Untersuchungssystem wird das distale Ende 12 einer bildgebenden Einrichtung 10 in einen Hohlraum 42 in einem Gehäuse 41 einer Prüfvorrichtung 40 eingeführt. Eine Positionierungseinrichtung 50 hält die bildgebende Einrichtung 10, insbesondere deren distales Ende 12, kraft- und/oder formschlüssig an einer vorbestimmten Position und in einer vorbestimmten Richtung relativ zu einer Referenzoberfläche 72. Die Referenzoberfläche ist insbesondere weiß bzw. weist einen innerhalb des für das menschliche Auge sichtbaren Spektralbereichs im Wesentlichen wellenlängenunabhängigen Remissionsgrad auf.

Die Referenzoberfläche 72 wird dann von einer Lichtquelle 22 mit Beleuchtungslicht beleuchtet. Neben den spektralen Eigenschaften der Lichtquelle 22 bestimmt ein Beleuchtungsfilter 24 im Beleuchtungs-Strahlengang das Spektrum des Beleuchtungslichts. Das Beleuchtungsfilter 24 kann manuell oder maschinell in den Beleuchtungs-Strahlengang eingeführt und aus diesem entfernt werden. In der Regel stehen mehrere Beleuchtungsfilter 24 zur Verfügung, die alternativ in den Beleuchtungs-Strahlengang eingeführt werden können. Die Lichtquelle 22 erzeugt beispielsweise ein vom menschlichen Auge als weiß wahrgenommenes Spektrum. Wenn kein Beleuchtungsfilter 24 im Beleuchtungs-Strahlengang angeordnet ist, wird die Referenzoberfläche 72 somit durch weißes Licht beleuchtet.

Zunächst wird ein für die vorgesehene Anwendung des optischen Untersuchungssystems geeignetes Beleuchtungsfilter 24 eingeführt. Wenn das optische Untersuchungssystem zur Betrachtung eines Objekts in Weißlicht vorgesehen ist und die Lichtquelle 22 weißes Licht erzeugt, wird kein Beleuchtungsfilter in den Beleuchtungs-Strahlengang eingeführt.

Bei Beleuchten der Referenzoberfläche 72 mit Beleuchtungslicht des vorgesehenen Spektrums kann ein Weißabgleich der Kamera 31 durchgeführt werden. Dieser Weißabgleich kann manuell oder automatisch erfolgen, wobei ein automatischer Weißabgleich manuell oder automatisch gesteuert bzw. ausgelöst werden kann. Beim Weißabgleich werden beispielsweise zwei Weißabgleich-Parameter bestimmt, die auch als WBGs (WBG = white balance gain) bezeichnet werden. Der erste Parameter WBGr bestimmt das Verhältnis der Verstärkungen im roten und im grünen Farbkanal, der zweite Parameter WBGb bestimmt das Verhältnis der Verstärkungen im blauen und im grünen Farbkanal. Die Verstärkungen sind analoge Verstärkungen vor einer Digitalisierung von im Bildsensor primär entstehenden elektrischen Signalen. Alternativ bestimmen die Weißabgleich-Parameter beispielsweise das Verhältnis von Korrekturfaktoren, die auf digitalisierte Signale der einzelnen Farbkanäle anzuwenden sind.

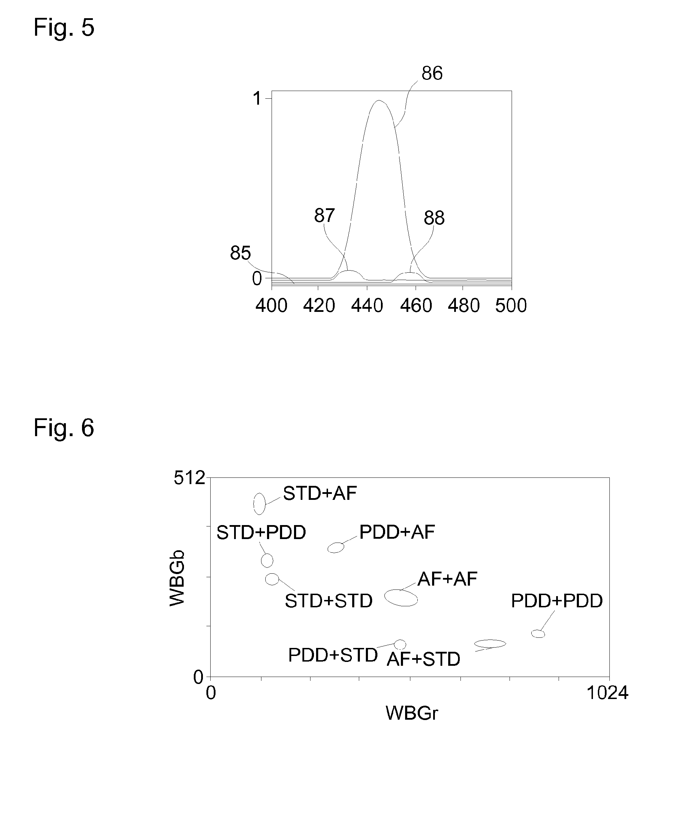

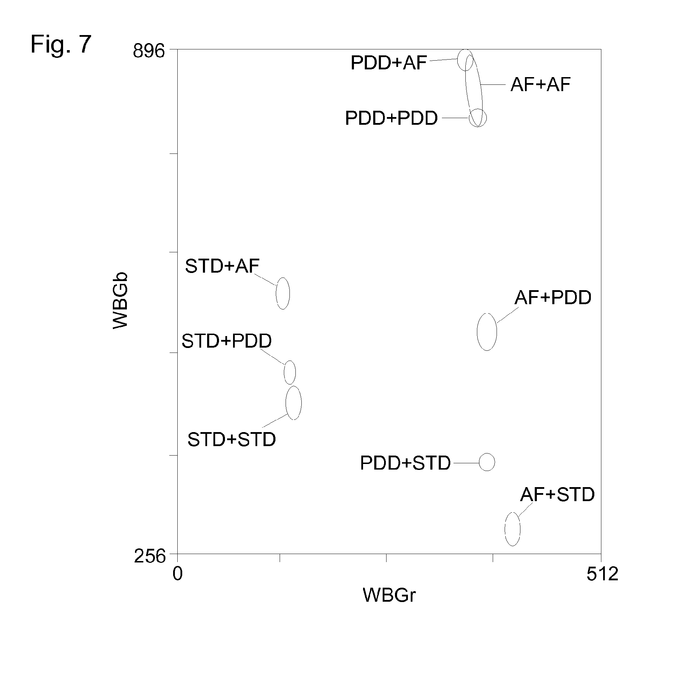

Der beschriebene Weißabgleich ist optional. Er ist jedoch in vielen Fällen erforderlich oder sinnvoll, um bei einer nachfolgenden Verwendung des optischen Untersuchungssystems einen natürlichen Farbeindruck zu erzielen. Die Weißabgleich-Parameter stellen einen Teil des Betriebszustands der Kamera 31 dar. Wenn der beschriebene Weißabgleich durchgeführt wird, kann aus den Werten der Weißabgleich-Parameter auf Eigenschaften des optischen Untersuchungssystems geschlossen werden. Dies wird unten mit Bezug auf die Figuren 6 und 7 näher beschrieben.

Wenn die Referenzoberfläche 72 nicht homogen weiß ist bzw. nicht an allen Orten einen für das menschliche Auge wellenlängenunabhängigen Remissionsgrad aufweist, können für den Weißabgleich manuell oder automatisch einer oder mehrere weiße Bereiche der Referenzoberfläche 72 ausgewählt werden. Wenn ein Weißabgleich an einer nicht-weißen Referenzoberfläche 72 oder an einem nicht-weißen Bereich der Referenzoberfläche 72 vorgenommen wird, kann aus den resultierenden Weißabgleich-Parametern ebenfalls auf Eigenschaften des optischen Untersuchungssystems geschlossen werden.

Bei Beleuchtung der Referenzoberfläche 72 mit dem vorgesehenen Beleuchtungslicht wird mittels der bildgebenden Einrichtung 10 ein Bild der Referenzoberfläche 72 erzeugt. Dieses Bild in von der Referenzoberfläche 72 remittierten Beleuchtungslicht und gegebenenfalls in von der Referenzoberfläche 72 emittiertem Fluoreszenzlicht wird von einem Beobachtungsfilter 13 spektral gefiltert. Das von der bildgebenden Einrichtung 10 erzeugte und gegebenenfalls vom Beobachtungsfilter 13 gefilterte Bild wird visuell erfasst bzw. über ein Okular 14 betrachtet oder von einer Kamera 31 erfasst. Bei der Erfassung des Bilds stellt die Kamera 31 selbst oder die Kamerasteuerung 35 einen Betriebszustand der Kamera 31 so ein, dass das erfasste Bild bzw. die analogen oder digitalen elektrischen Signale vorbestimmten Bedingungen entsprechen. Zu diesen Bedingungen zählt beispielsweise ein vorbestimmter Mittelwert der Helligkeitswerte im gesamten erfassten Bild oder in einem Teilbereich des erfassten Bilds. Parameter, die den Betriebszustand der Kamera 31 bilden bzw. beschreiben und typischerweise in Abhängigkeit von der Helligkeit des von der bildgebenden Einrichtung 10 am Bildsensor der Kamera 31 erzeugten optischen Bilds eingestellt werden, sind die Belichtungszeit und die (insbesondere analoge) Verstärkung der primären elektrischen Signale vor ihrer Digitalisierung. Sowohl die Belichtungszeit als auch die Verstärkung können für alle Bildpunkte und alle Farbkanäle die gleichen Werte oder für verschiedene Farbkanäle oder verschiedene Bereiche des Bildsensors verschiedene Werte aufweisen.

Bei Beleuchten der Referenzoberfläche 72 werden ein oder mehrere Parameter des automatisch eingestellten Betriebszustands der Kamera 31 erfasst, beispielsweise durch Abfragen oder Auslesen aus einem Speicher. Anhand der erfassten Betriebsparameter der Kamera 31 kann auf die Belichtungssituation, insbesondere die Helligkeit, des von der bildgebenden Einrichtung 10 am Bildsensor der Kamera 31 erzeugten optischen Bilds geschlossen werden. Für diese Helligkeit existieren Mindestwerte, die von der vorgesehenen Anwendung des optischen Untersuchungssystems abhängig sein können. Bei vollständig richtiger Konfiguration des optischen Untersuchungssystems, insbesondere wenn alle Komponenten uneingeschränkt funktionsfähig und fehlerfrei miteinander verbunden bzw. gekoppelt sind und sowohl Beleuchtungs- als auch Beobachtungsfilter der vorgesehenen Verwendung entsprechen, erfüllt das von der bildgebenden Einrichtung 10 am Bildsensor der Kamera 31 erzeugte optische Bild die Anforderungen und ist insbesondere ausreichend hell.

Wenn der Betriebszustand der Kamera 31 eine vorbestimmte Bedingung erfüllt (beispielsweise in der oben beschriebenen Stufe 1 zu liegen) kann auf eine uneingeschränkte Funktionsfähigkeit des optischen Untersuchungssystems geschlossen werden. Wenn der erfasste Betriebszustand der Kamera 31 der vorbestimmten Bedingung nicht entspricht (beispielsweise in einer der oben beschriebenen Stufen 2 und 3 liegt), wird daraus geschlossen, dass das optische Untersuchungssystem nicht oder nicht uneingeschränkt funktionsfähig ist. Beispielsweise liefert die Lichtquelle 22 einen zu geringen Lichtstrom, das Lichtleitkabel 19 ist defekt oder nicht einwandfrei mit der Lichtquelle 22 oder der bildgebenden Einrichtung 10 gekoppelt, die bildgebende Einrichtung 10 ist defekt, ein falsches Beleuchtungsfilter ist im Beleuchtungs-Strahlengang angeordnet oder ein falsches Beobachtungsfilter ist im Beobachtungs-Strahlengang angeordnet.

Zu einem beliebigen Zeitpunkt vor dem Vergleichen des erfassten Betriebszustands der Kamera 31 mit der vorbestimmten Bedingung kann die Anwendung, für die das optische Untersuchungssystem vorgesehen ist, erfasst werden. Dazu wird insbesondere eine Eingabe an einer Benutzerschnittstelle nach einer entsprechenden Aufforderung erfasst. Da verschiedene Anwendungen des optischen Untersuchungssystems unterschiedliche Anforderungen an die Qualität der erfassten Bilder stellen, können verschiedenen Anwendungen verschiedene Bedingungen an den erfassten Betriebszustand zugeordnet sein. Die Bedingung, der der erfasste Betriebszustand des optischen Untersuchungssystems genügen muss, wird als die der erfassten vorgesehenen Anwendung zugeordnete Bedingung ermittelt. Die Bedingung umfasst beispielsweise einen oder mehrere Schwellenwerte für den Betriebszustand, insbesondere Schwellenwerte für die Belichtungszeit, für die Verstärkung oder für einen daraus berechneten Belichtungsparameter.

Nach der beschriebenen Bestimmung der Funktionsfähigkeit des Untersuchungssystems für die vorgesehene Anwendung kann eine entsprechende Meldung über eine Benutzerschnittstelle ausgegeben werden. Diese Meldung kann Handlungsanweisungen oder Handlungsempfehlungen umfassen. Beispielsweise wird, wenn der Betriebszustand die Bedingung nicht erfüllt, eine Aufforderung zur Prüfung der Komponenten des optischen Untersuchungssystems und ihrer Kopplung oder zum Austausch der Lichtquelle 22 oder einer anderen Komponente und zur Wiederholung des Prüfverfahrens ausgegeben.

Die Genauigkeit des beschriebenen Prüfverfahrens kann beispielsweise erhöht werden, indem aus dem Betriebszustand der Kamera, insbesondere der Belichtungszeit, der Verstärkung und/oder weiteren Parametern, ein Belichtungsparameter berechnet wird. Der Belichtungsparameter E wird beispielsweise nach der bereits erwähnten Formel E=a·T^{b}·G^{c} berechnet. Anhand des Belichtungsparameters können eine detaillierte Aussage über die Funktionsfähigkeit des optischen Untersuchungssystems und eine präzisere Handlungsempfehlung ausgesprochen werden. Beispielsweise kann danach unterschieden werden, ob das optische Untersuchungssystem für die vorgesehene Anwendung uneingeschränkt, eingeschränkt oder überhaupt nicht tauglich ist. Der Belichtungsparameter kann abgelegt bzw. gespeichert werden. Bei wiederholter Prüfung des gleichen optischen Untersuchungssystems kann ein Trend bzw. eine zeitliche Entwicklung des Belichtungsparameters ermittelt werden, der beispielsweise auf einen Alterungsprozess der Lichtquelle 22 zurückgeführt werden kann. Aus dem Stadium des Alterungsprozesses der Lichtquelle 22 wird beispielsweise eine Empfehlung zu deren Austausch oder zu Einschränkungen im Betrieb der Lichtquelle 22 gegeben.

Im Rahmen des beschriebenen Prüfverfahrens können, insbesondere über eine Benutzerschnittstelle, Patientendaten erfasst und anschließend gemeinsam mit dem Ergebnis des Prüfverfahrens und insbesondere zusammen mit dem Ergebnis einer nachfolgenden Untersuchung des Patienten mittels des optischen Untersuchungssystems in einer Datenbank abgelegt werden. Damit wird sichergestellt, dass das optische Untersuchungssystem vor oder nach der Untersuchung eines Patienten auf seine Funktionsfähigkeit geprüft und das Ergebnis dieser Prüfung protokolliert bzw. dokumentiert wird.

Die den verschiedenen Anwendungen des optischen Untersuchungssystems zugeordneten Bedingungen bzw. Schwellenwerte für den Betriebszustand oder für die den Betriebszustand charakterisierenden Parameter können empirisch bestimmt werden, indem medizinisches Personal Untersuchungen, die der vorgesehenen Verwendung entsprechen, unter realen Bedingungen aber bei unterschiedlichen Beleuchtungssituationen vornehmen und die Qualität des Bilds des betrachteten Objekts bewerten.

Ein bei einer vorgesehenen Anwendung des optischen Untersuchungssystems zu betrachtendes Objekt kann einen Remissionsgrad und andere optische Eigenschaften aufweisen, die sich von denen der Referenzoberfläche 72 unterscheiden. Dem kann während des oben beschriebenen Prüfverfahren dadurch Rechnung getragen werden, dass die Helligkeit der Lichtquelle 22 während des Prüfverfahrens verändert bzw. angepasst wird, beispielsweise durch Dimmen oder durch Verwendung einer Blende, eines Gitters oder eines Filters. Insbesondere wird der zur Verfügung stehende Lichtfluss um einen Faktor reduziert, der dem Verhältnis zwischen dem Remissionsgrad eines bei der vorgesehenen Verwendung des optischen Untersuchungssystems relevanten Objekts und dem Remissionsgrad der Referenzoberfläche 72 entspricht.

Wenn die verwendete Kamera 31 dies zulässt, kann bei dem oben beschriebenen Prüfverfahren beispielsweise entweder die Belichtungszeit oder die Verstärkung fest vorgegeben werden und lediglich der sich dabei einstellende jeweils andere Parameter erfasst und entsprechend der obigen Beschreibung ausgewertet werden.

Nachfolgend werden weitere Varianten des oben beschriebenen Prüfverfahrens beschrieben, die beispielsweise anwendbar sind, wenn die vorgesehene Anwendung des optischen Untersuchungssystems PDD, AF-Diagnostik oder eine andere Fluoreszenz-Diagnostik ist. Zur Erläuterung werden zunächst Fluoreszenz-Anregungs- und -Abregungsspektren sowie Transmissionsspektren von Beleuchtungs- und Beobachtungsfiltern für die Fluoreszenz-Diagnostik beschrieben. Beispielsweise die zur PDD und die zur AF-Diagnostik verwendeten Filter unterscheiden sich, sind jedoch bei visueller Betrachtung leicht verwechselbar. Das oben beschriebene Prüfverfahren kann so modifiziert werden, dass die verwendeten Filter identifiziert werden können.

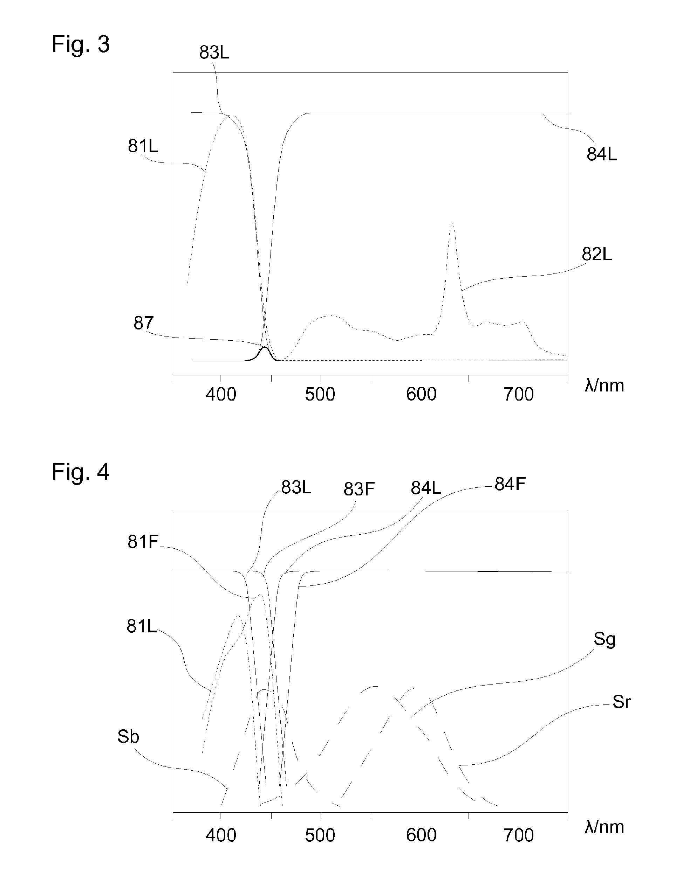

Figur 3 zeigt eine schematische Darstellung eines Fluoreszenz-Anregungsspektrums 81L und eines Fluoreszenz-Abregungsspektrums 82L von durch 5-Aminolävulinsäure (ALA) induzierter Fluoreszenz von Protoporphyrin IX. Der Abszisse ist die Wellenlänge λ zugeordnet, der Ordinate die Quantenausbeute bzw. die Intensität in willkürlichen Einheiten. Ferner sind ein Transmissionsspektrum 83L eines geeigneten Beleuchtungsfilters 24 und ein Transmissionsspektrum 84L eines geeigneten Beobachtungsfilters 13 dargestellt. Für die Transmissionsspektren 83L und 84L ist der Ordinate jeweils der Transmissionsgrad zugeordnet.

Ferner ist das Produkt 87 aus den Transmissionsspektren 83L, 84L bzw. das Transmissionsspektrum der hintereinander geschalteten Beleuchtungs- und Beobachtungsfilter dargestellt. Die Filterkanten des Beleuchtungsfilters 24 und des Beobachtungsfilters 23 sind so gewählt, dass das Produkt aus deren Transmissionsspektren in einem kleinen Wellenlängenbereich nicht 0 ist, der auch als Überlappbereich bezeichnet wird. Ein kleiner Anteil des auf das beobachtete Objekt treffenden Beleuchtungslichts kann deshalb durch das Beobachtungsfilter 13 beobachtet werden. Das beobachtete Objekt ist deshalb auch ohne Fluoreszenz in (ohne Wellenlängenverschiebung) remittiertem blauem Beleuchtungslicht erkennbar. Die Fluoreszenz erscheint demgegenüber überwiegend im grünen und roten Spektralbereich. Damit liegt zwischen fluoreszierenden und nicht-fluoreszierenden Bereichen eines mittels des optischen Untersuchungssystems beobachteten Objekts ein deutlicher Farbkontrast vor.

Figur 4 zeigt eine schematische Darstellung von Fluoreszenz-Anregungsspektren sowie Transmissionsspektren von Beleuchtungs- und Beobachtungsfiltern, die für verschiedene Arten der Fluoreszenz-Diagnostik verwendet werden. Der Abszisse ist die Wellenlänge λ zugeordnet. Gezeigt sind neben dem Fluoreszenz-Anregungsspektrum 81L, dem Transmissionsspektrum 83L des Beleuchtungsfilters und dem Transmissionsspektrum 84L des Beobachtungsfilters für PDD auch das Fluoreszenz-Anregungsspektrum 81F, das Transmissionsspektrum 83F des Beleuchtungsfilters und das Transmissionsspektrum 84F des Beobachtungsfilters zur Beobachtung von Autofluoreszenz (AF) von Gewebe.

In Figur 4 sind ferner die spektralen Empfindlichkeiten Sb, Sg, Sr der blauen, grünen und roten Farbrezeptoren des menschlichen Auges dargestellt. Da Kameras möglichst weitgehend an das Farbempfinden des menschlichen Auges angepasst werden, weisen sie in der Regel ähnliche spektrale Empfindlichkeiten auf oder trennen die Farbkanäle noch schärfer. Im Vergleich der Transmissionsspektren 83L, 83F, 84L, 84F der Beleuchtungs- und Beobachtungsfilter für PDD und AF mit den spektralen Empfindlichkeiten der Farbrezeptoren des menschlichen Auges wird deutlich, dass die geringen Unterschiede zwischen den Transmissionsspektren der Beleuchtungs- und Beobachtungsfilter für PDD und AF für das menschliche Auge nur unter guten Bedingungen im unmittelbaren Vergleich - der selten möglich ist - erkennbar sind.

Figur 5 zeigt eine schematische Darstellung verschiedener Produkte jeweils eines Transmissionsspektrums eines Beleuchtungsfilters und eines Transmissionsspektrums eines Beobachtungsfilters. Die Kurven sind vertikal geringfügig gegeneinander verschoben, damit sie leichter unterschieden werden können. Tatsächlich sind alle Produkte bei Wellenlängen um 400 nm und bei Wellenlängen um 500 nm näherungsweise 0.

Das Produkt 85 aus dem Transmissionsspektrum 83L des PDD-Beleuchtungsfilters und dem Transmissionsspektrum 84F des AF-Beobachtungsfilters ist für alle Wellenlängen sehr klein bzw. näherungsweise 0. Das AF-Beobachtungsfilter ist somit für remittiertes PDD-Anregungslicht nicht transparent.

Das Produkt 86 aus dem Transmissionsspektrum 83F des Beleuchtungsfilters für AF-Diagnostik und dem Transmissionsspektrum 84L des Beobachtungsfilters für PDD ist für Wellenlängen im Bereich von ca. 430 nm bis ca. 460 nm deutlich größer als 0. Das PDD-Beobachtungsfilter ist für remittiertes AF-Anregungslicht somit in einem deutlich sichtbaren Maß transparent.

Das Produkt 87 aus dem Transmissionsspektrum 83L des Beleuchtungsfilters für PDD und dem Transmissionsspektrum 84L des Beobachtungsfilters für PDD ist, wie bereits oben anhand der Figur 4 dargestellt, in einem kleinen Wellenlängenbereich zwischen ca. 430 nm und ca. 440 nm nicht 0. Das PDD-Beobachtungsfilter ist für remittiertes PDD-Anregungslicht geringfügig transparent.

Das Produkt 88 aus dem Transmissionsspektrum 83F des Beleuchtungsfilters für AF und dem Transmissionsspektrum 84F des Beobachtungsfilters für AF ist in einem kleinen Wellenlängenbereich in der Gegend von 460 nm nicht 0. Das AF-Beobachtungsfilter ist für remittiertes AF-Anregungslicht geringfügig transparent.

Bei Betrachtung einer weißen, nicht fluoreszierenden Referenzoberfläche mit einem optischen Untersuchungssystem kann somit unter günstigen Umständen unterscheidbar sein, ob ein PDD-Beleuchtungsfilter mit einem AF-Beobachtungsfilter oder ein AF-Beleuchtungsfilter mit einem PDD-Beobachtungsfilter kombiniert ist. Im ersten Fall wird ein extrem dunkles Bild beobachtet, im zweiten Fall ein im Vergleich zu korrekten Kombinationen von Beleuchtungsfilter und Beobachtungsfilter zu helles Bild. Kaum unterscheidbar hingegen ist, ob ein Beleuchtungsfilter für PDD mit einem Beobachtungsfilter für PDD oder ein Beleuchtungsfilter für AF mit einem Beobachtungsfilter für AF kombiniert ist. In beiden Fällen ist das Bild näherungsweise gleich hell, der Unterschied in der Wellenlänge ist für das menschliche Auge allenfalls unter sehr guten Bedingungen im unmittelbaren Vergleich unterscheidbar.

Das oben beschriebene Prüfverfahren kann derart modifiziert werden, dass als weitere Eigenschaft des optischen Untersuchungssystems das Beleuchtungsfilter und das Beobachtungsfilter identifiziert werden können. Die nachfolgende Tabelle zeigt den Belichtungsparameter E für alle möglichen Kombinationen einer Weißlicht- bzw. Standard-Beleuchtung (STD; erste Zeile), eines PDD-Beleuchtungsfilters (zweite Zeile) oder eines AF-Beleuchtungsfilters (dritte Zeile) mit einem Standard-Endoskop für Beobachtung in remittiertem Weißlicht (erste Spalte), einem PDD-Endoskop (zweite Spalte) oder einem AF-Endoskop (dritte Spalte). Das Standard-Endoskop weist sowohl im Beleuchtungs- als auch im Beobachtungs-Strahlengang eine im für das menschliche Auge sichtbaren Wellenlängenbereich möglichst weitgehend wellenlängenunabhängige bzw. dem menschlichen Auge als im Wesentlichen nicht farbstichig erscheinende Transmission auf. Das PDD-Endoskop weist im Beobachtungs-Strahlengang ein Beobachtungsfilter mit den oben anhand der Figur 4 dargestellten Transmissionsspektrum 84L auf, oder der Beobachtungs-Strahlengang weist ohne dediziertes Beobachtungsfilter eine entsprechende Filtercharakteristik auf. Das AF-Endoskop weist im Beobachtungs-Strahlengang ein Beobachtungsfilter mit dem oben anhand der Figur 4 dargestellten Transmissionsspektrum 84F auf, oder der Beobachtungs-Strahlengang des AF-Endoskops weist ohne ein dediziertes Beobachtungsfilter eine entsprechende Filtercharakteristik auf.

| E | STD-Endoskop | PDD-Endoskop | AF-Endoskop |
|---|---|---|---|
| STD-Beleuchtung | 0,0023 | 0,0030 | 0,0033 |
| PDD-Beleuchtung | 0,0020 | 2,50 | "∞" |
| AF-Beleuchtung | 0,0014 | 0,0344 | 2,50 |

Ein in vielen Situationen deutlich erkennbarer Unterschied liegt vor zwischen einer Kombination aus PDD-Beleuchtung und AF-Endoskop (Belichtungsparameter E ist sehr groß bzw. unendlich) einerseits und den zulässigen Kombinationen aus PDD-Beleuchtung und PDD-Endoskop bzw. aus AF-Beleuchtung und AF-Endoskop (in beiden Fällen beträgt der Belichtungsparameter ca. 2,5) andererseits. Ein in der Regel deutlich erkennbarer Unterschied liegt auch zwischen den zulässigen Kombinationen aus PDD-Beleuchtung und PDD-Endoskop bzw. aus AF-Beleuchtung und AF-Endoskop einerseits und den anderen Kombinationen vor, bei denen der Belichtungsparameter Werte annimmt, die deutlich kleiner als 1 sind. Die genannten Zahlen sind jedoch nur Beispiele, die bei einem individuellen optischen Untersuchungssystem gemessen wurden. Nach jeder Modifikation des optischen Untersuchungssystems oder an anderen optischen Untersuchungssystemen können davon abweichende Werte des Belichtungsparameters E gemessen werden.

Für ein optisches Untersuchungssystem mit einem bestimmten Endoskop (entweder Standard-Endoskop oder PDD-Endoskop oder AF-Endoskop) werden nun drei verschiedene Belichtungsparameter E für drei verschiedene Spektren des Anregungs- bzw. Beleuchtungslichts (weiß bzw. Standard STD; PDD; AF) ermittelt und Quotienten der Belichtungsparameter E gebildet. Diese Quotienten sind in der nachfolgenden Tabelle wiedergegeben.

| | STD-Endoskop | PDD-Endoskop | AF-Endoskop |
|---|---|---|---|
| E_{PDD}/E_{STD} | 0,84 | 832 | "∞" |
| E_{AF}/E_{STD} | 0,61 | 11,5 | 755 |
| E_{PDD}/E_{AF} | 1,38 | 72,6 | "∞" |

Es ist erkennbar, dass die drei verschiedenen Endoskope in jedem der drei Quotienten des Belichtungsparameters E unterscheidbar sind. Durch die Quotientenbildung wird ferner der Einfluss weiterer Komponenten des optischen Untersuchungssystems, insbesondere des Lichtleitkabels 19, seiner Kopplung an die Lichtquelle 22 und das Endoskop 10, der Abstand von der Referenzoberfläche 72 etc., unterdrückt. Damit entfällt auch die Notwendigkeit einer genauen Positionierung des distalen Endes 12 des Endoskops bzw. der bildgebenden Einrichtung relativ zur Referenzoberfläche 72.

Das oben beschriebene Prüfverfahren kann zur Identifizierung des im zu prüfenden optischen Untersuchungssystem verwendeten Endoskops (bzw. einer entsprechenden bildgebenden Einrichtung) wie folgt modifiziert werden. Nacheinander werden Bilder der Referenzoberfläche 72 mit zwei verschiedenen Beleuchtungsspektren erfasst, beispielsweise bei Weißlicht-Beleuchtung bzw. ohne Beleuchtungsfilter und bei einem PDD-Beleuchtungsfilter mit dem oben anhand der Figur 4 dargestellten Transmissionsspektrum 83L im Beleuchtungs-Strahlengang. Für beide erfassten Bilder werden die dabei vorliegenden Betriebszustände der Kamera 31 erfasst und aus diesen je ein Belichtungsparameter E_{STD} bzw. E_{PDD} nach der bereits erwähnten Formel E=a·T·G berechnet. Schließlich wird geprüft, ob der Quotient E_{STD}/E_{PDD} näher bei dem für ein Standard-Endoskop nötigen Wert (ca. 1) oder näher bei dem für ein PDD-Endoskop gültigen Wert (ca. 800) liegt oder noch wesentlich größer ist. Wenn der Wert des Quotienten E_{STD}/E_{PDD} wesentlich größer als 800 ist, umfasst das optische Untersuchungssystem mit hoher Wahrscheinlichkeit ein AF-Endoskop, wenn der Quotient E_{STD}/E_{PDD} in der Nähe von 800 liegt, liegt ein PDD-Endoskop vor und wenn der Quotient E_{STD}/E_{PDD} bei 1 liegt, liegt ein Standard-Endoskop vor.

Das beschriebene Verfahren ist besonders vorteilhaft und vor allem besonders schnell durchführbar, wenn die Lichtquellenvorrichtung 20 des optischen Untersuchungssystems einen maschinellen Wechsel des Beleuchtungsfilters 24 vorsieht. Das Verfahren ist jedoch beispielsweise auch mit einem manuellen Wechsel des Beleuchtungsfilters 24 oder einem entsprechenden Austausch von kompletten Lichtquellenvorrichtungen 20 mit jeweils fest installiertem Beleuchtungsfiltern bzw. unveränderlichen Beleuchtungsspektren durchführbar.

Entsprechend kann in einem optischen Untersuchungssystem das Beleuchtungsfilter bzw. das Beleuchtungsspektrum identifiziert werden. Zunächst werden Bilder der Referenzoberfläche mit zwei verschiedenen Endoskopen bzw. bei zwei verschiedenen Beobachtungsfiltem erfasst. Dabei wird jeweils der vorliegende Betriebszustand der Kamera erfasst. Aus den Betriebszuständen werden zwei Belichtungsparameter berechnet. Anhand des Quotienten aus den beiden Belichtungsparametern kann das Beleuchtungsfilter identifiziert werden.

Obwohl bei realen Endoskopiesystemen und anderen optischen Untersuchungssystemen die Werte der Belichtungsparameter und ihre Quotienten streuen, zeigen die dargestellten Modellrechnungen, dass das Verfahren zur Identifizierung des Beobachtungsfilters bzw. des Beleuchtungsfilters geeignet ist. Insbesondere ist die Streuung der gemessenen Quotienten Eₓ/E_{y} deutlich kleiner als die Abstände der typischen Werte der Quotienten Eₓ/E_{y}. Dadurch ist das Verfahren sehr robust.

Nachfolgend wird eine weitere Variante des Prüfverfahrens beschrieben, mit dem das Beobachtungsfilter und/oder das Beleuchtungsfilter identifiziert werden kann. Dieses Verfahren ist insbesondere durchführbar, wenn der Betriebszustand der Kamera 31 nach Farbkanälen differenziert erfasst werden kann, also beispielsweise für jeden Farbkanal die Belichtungszeit und/oder die Verstärkung separat erfasst werden können. Das Verfahren ist jedoch auch durchführbar, wenn der Betriebszustand bzw. die Belichtungszeit und die Verstärkung nicht für jeden Farbkanal separat eingestellt werden und erfassbar sind, sondern beispielsweise nur für alle Farbkanäle gemeinsam und gleich eingestellt werden.

Bei dieser Variante des Prüfverfahrens stellt die Kamera bzw. die Kamerasteuerung selbsttätig Belichtungszeit, Verstärkung oder andere Parameter ein, oder diese Parameter werden von außen vorgegeben. Aus der Kamera bzw. der Kamerasteuerung werden anschließend für jeden Farbkanal ein Akkumulatorwert für das erfasste Bild ausgelesen. Der Akkumulatorwert A_{b}, Ag, Aᵣ in einem Farbkanal b, g, r wird beispielsweise als Summe der Intensitätswerte, die den einzelnen Bildpunkten innerhalb eines vorbestimmten Bereichs des Bilds für den betreffenden Farbkanal zugeordnet sind, berechnet.

Die nachfolgende Tabelle zeigt in jedem Feld untereinander den Akkumulatorwert A_{b} für den blauen Farbkanal, den Akkumulatorwert Ag für den grünen Farbkanal und den Akkumulatorwert Aᵣ für den roten Farbkanal.

| A_{b} | | | |
|---|---|---|---|
| A_{g} | | | |
| Aᵣ | STD-Endoskop | PDD-Endoskop | AF-Endoskop |
| STD-Beleuchtung | 728 | 497 | 227 |
| | 804 | 992 | 1070 |
| | 776 | 965 | 1064 |
| PDD-Beleuchtung | 857 | 3549 | 0 |
| | 1 | 109 | 0 |
| | 0 | 0 | 0 |
| AF-Beleuchtung | 1862 | 3497 | 3146 |
| | 40 | 125 | 236 |
| | 0 | 0 | 0 |

Für jede der neun möglichen Kombinationen von einem der drei Beleuchtungsspektren mit einem der drei Endoskope wird der Quotient (Ag+Aᵣ)/A_{b} aus der Summe des Akkumulatorwert A_{g} für den gründen Farbkanal und des Akkumulatorwert Aᵣ für den roten Farbkanal sowie dem Akkumulatorwert A_{b} für den blauen Farbkanal gebildet. Diese Quotienten sind in der nachfolgenden Tabelle angegeben.

| (A_{g}+Aᵣ)/A_{b} | STD | PDD | AF |
|---|---|---|---|
| STD-Beleuchtung | 2,17 | 3,94 | 9,40 |
| PDD-Beleuchtung | 0,001 | 0,031 | "∞" |
| AF-Beleuchtung | 0,022 | 0,036 | 0,075 |

Es ist erkennbar, dass bereits mit einem einzigen Beleuchtungsspektrum anhand des Quotienten (A_{g}+Aᵣ)/A_{b} unterschieden werden kann, ob das optische Untersuchungssystem ein Standard-Endoskop, ein PDD-Endoskop oder ein AF-Endoskop umfasst. Dazu wird beispielsweise das oben beschriebene Prüfverfahren wie folgt abgewandelt. Bei Weißlichtbeleuchtung der Referenzoberfläche 72 bzw. ohne Beleuchtungsfilter 24 wird ein Bild der Referenzoberfläche 72 erfasst. Für jeden der drei Farbkanäle b, g, r wird bei gegebener Belichtungszeit und Verstärkung die Bildhelligkeit oder die Helligkeit eines Teilbilds, wiedergegeben durch einen Akkumulatorwert A_{b}, Ag, Aᵣ erfasst. Aus den Akkumulatorwert A_{b}, Ag, Aᵣ wird der Quotient (A_{g}+Aᵣ)/A_{b} berechnet. Wenn der Wert dieses Quotienten (A_{g}+Aᵣ)/A_{b} in der Umgebung von 2 liegt, umfasst das optische Untersuchungssystem ein Standard-Endoskop, wenn der Quotient in der Umgebung von 4 liegt, ein PDD-Endoskop und wenn der Quotient in der Nähe von 9 liegt, ein AF-Endoskop.

Anhand der Tabelle ist ferner erkennbar, dass durch Ermittlung des Quotienten Q1=(A_{g}+Aᵣ)/A_{b} für Standard-Beleuchtung und des Quotienten Q2=(A_{g}+Aᵣ)/A_{b} für PDD-Beleuchtung und Division der beiden so gewonnenen Quotienten Q1 und Q2 ebenfalls das im optischen Untersuchungssystem vorliegende Endoskop identifiziert werden kann. Wenn das Verhältnis Q1/Q2 der Quotienten für Standard-Beleuchtung und für PDD-Beleuchtung bei 2000 liegt, liegt ein Standard-Endoskop vor, wenn das Verhältnis Q1/Q2 der Quotienten bei 100 liegt, liegt ein PDD-Endoskop vor, und wenn das Verhältnis Q1/Q2 der Quotienten näherungsweise 0 ist, liegt ein AF-Endoskop vor.

Wenn der Betriebszustand der Kamera 31 nach Farbkanälen differenziert erfasst werden kann, also beispielsweise für jeden Farbkanal die Belichtungszeit und/oder die Verstärkung verschiedene Werte annehmen und separat erfasst werden kann, kann ein entsprechendes Verfahren mit den Belichtungsparametern E_{b}, E_{g}, Eᵣ durchgeführt werden, die den einzelnen Farbkanälen zugeordnet sind. In den oben genannten Formeln wird jeweils A_{b} durch E_{b}, Ag durch Eg und Aᵣ durch Eᵣ ersetzt.

Bei realen endoskopischen oder anderen optischen Untersuchungssystemen variieren bzw. streuen die Werte der Belichtungsparameter E und der daraus gebildeten Quotienten. Dennoch können durch diese und ähnliche algebraische Verknüpfungen oder auch durch logische Verknüpfung von Belichtungsparametern Beobachtungsfilter und/oder Beleuchtungsfilter identifiziert werden.

Der Betriebszustand einer Kamera 31 wird unter anderem auch durch die Weißabgleich-Parameter bestimmt. Nachfolgend beschriebene Varianten der oben beschriebenen Prüfverfahren sind auf Weißabgleich-Parameter als Parameter des Betriebszustands der Kamera gestützt.

Wie bereits erwähnt, dient der Weißabgleich der Erzielung eines natürlichen Farbeindrucks. Zum Weißabgleich wird ein Bild einer weißen oder grauen Fläche erfasst. Zum Weißabgleich ist insbesondere die oben beschriebene Referenzoberfläche 72 der Prüfvorrichtung 40 geeignet, wenn sie weiß ist bzw. einen im für das menschliche Auge sichtbaren Spektralbereich im Wesentlichen wellenlängenunabhängigen Remissionsgrad aufweist. Beim Weißabgleich werden die Signale, insbesondere die digitalen Signale, in den drei Farbkanälen miteinander verglichen. Es werden Gewichtsfaktoren für die Farbkanäle berechnet, so dass das Produkt aus Rohsignal und Gewichtsfaktor für jeden Farbkanal gleich ist. Anstelle eines auf das digitale Signal anzuwendenden Gewichtsfaktors können die Belichtungszeiten oder die Verstärkungen um entsprechende Faktoren verändert werden.

Da nur zwei Freiheitsgrade erforderlich sind, um einen Weißabgleich durchzuführen, wird einer der Gewichtsfaktoren bzw. Weißabgleich-Parameter WBGb, WBGg, WBGr nicht verändert. Gemäß einer weit verbreiteten Konvention beträgt der Weißabgleich-Parameter WBGg immer WBGg = 128 = Ox080. Zur Kompensation einer Beleuchtung mit übermäßigem Blauanteil wird beispielsweise ein Weißabgleich-Parameter WBGb < 128 für den blauen Farbkanal eingestellt. Zur Kompensation einer Beleuchtung mit übermäßigem Rotanteil wird ein Weißabgleich-Parameter WBGr < 128 eingestellt.

Bei fluoreszenzdiagnostischen Verfahren, wie der PDD und der AF-Diagnostik wird der Weißabgleich genutzt, um trotz der verwendeten Beleuchtungs- und Beobachtungsfilter einen näherungsweise natürlichen Farbeindruck zu erzeugen. Wegen der gegenüber Weißlichtbeleuchtung reduzierten Gesamttransmission des Beleuchtungsfilters im Blaukanal ist es offensichtlich, dass dabei Weißabgleich-Parameter WBGb, WBGr eingestellt werden, die sich von den Weißabgleich-Parametern bei Weißlichtbeleuchtung und ohne Beobachtungsfilter deutlich unterscheiden. Wegen der oben anhand der Figur 4 dargestellten Unterschiede zwischen den Beleuchtungsfiltern und zwischen den Beobachtungsfiltern für die PDD und für die AF-Diagnostik unterscheiden sich aber auch die Weißabgleich-Parameter bei diesen beiden fluoreszenzdiagnostischen Verfahren von einander. Wiederum andere Weißabgleich-Parameter erhält man bei Weißabgleich einer Kamera 31 in einem optischen Untersuchungssystem mit weiteren Filterkombinationen.

Figur 6 zeigt in einem schematischen Diagramm typische Weißabgleich-Parameter nach einem Weißabgleich an einer weißen, nicht fluoreszierenden Referenzoberfläche bei verschiedenen Kombinationen von Beleuchtungsfiltern und Beobachtungsfiltern. Die Referenzoberfläche ist in diesem Beispiel eine Oberfläche eines Referenzkörpers aus weißem PTFE. Der Abszisse ist der bereits erwähnte Weißabgleich-Parameter WBGr, der Ordinate der Weißabgleich-Parameter WBGb zugeordnet. Bei den Messpunkten ist die Filterkombination jeweils angegeben, wobei die Angabe vor dem Pluszeichen das Beleuchtungsfilter und die Angabe nach dem Pluszeichen das Beobachtungsfilter angibt. Dabei bedeutet "STD" kein Filter (Weißlicht), "PDD" ein Filter für PDD und "AF" ein Filter für AF. Zulässige Filterkombinationen sind STD+STD, PDD+PDD und AF+AF.

Es ist erkennbar, dass verschiedene Filterkombinationen verschiedene Weißabgleich-Parameter zur Folge haben, die eindeutig zugeordnet und unterschieden werden können. Nach Durchführung eines Weißabgleichs mit einer geeigneten Referenzoberfläche kann somit aus dem Weißabgleich-Parameter auf die vorliegende Filterkombination geschlossen werden.

Da die Weißabgleich-Parameter von Kameratyp zu Kameratyp und in manchen Fällen sogar von Kamera zu Kamera variieren, können die bei einem Weißabgleich gewonnen Weißabgleich-Parameter beispielsweise durch in der Kamera abgelegte Korrektur-Parameter korrigiert werden. Die in der Kamera abgelegten Korrektur-Parameter sind beispielsweise ohne Beleuchtungs- und Beobachtungsfilter an einer weißen Fläche gewonnene Weißabgleich-Parameter. Diese Korrektur-Parameter können statt in der Kamera 31 auch in der Kamerasteuerung 35 abgelegt sein. Weitere Korrekturen können für die Bauform oder den Typ des Endoskops erfolgen, da starre und flexible Endoskope, Endoskope mit unterschiedlichen Durchmessern oder für unterschiedliche Anwendungen unterschiedliche Transmissionsspektren im Beleuchtungs-Strahlengang und im Beobachtungs-Strahlengang aufweisen.

Bei Verwendung einer nicht-weißen Referenzoberfläche für einen Weißabgleich kann die Genauigkeit bzw. Zuverlässigkeit der Unterscheidung verschiedener Filterkombinationen anhand der Weißabgleich-Parameter weiter verbessert werden. Dies gilt insbesondere, wenn Bereiche der Referenzoberfläche Stoffe aufweisen, deren Absorptions- oder Fluoreszenzanregungsspektren Kanten bzw. Flanken in der Nähe der Filterkanten der zu unterscheidenden Beleuchtungs- und Beobachtungsfilter aufweisen.

Figur 7 zeigt in einem schematischen Diagramm Weißabgleich-Parameter für verschiedene Filterkombinationen bei einem Weißabgleich auf eine Referenzoberfläche aus einer deckenden Schicht der von Marabu vertriebenen Farbe Maragloss GO 320 Fluoresco-Gelb. Abszisse und Ordinate sowie Kennzeichnung der Messwerte entsprechen denen aus Figur 6. Ein Vergleich der Figuren 6 und 7 zeigt, dass eine besonders sichere Identifikation der vorliegenden Filterkombination möglich ist, wenn sowohl die Weißabgleich-Parameter aus einem Weißabgleich an einer Teflonoberfläche als auch die Weißabgleich-Parameter aus einem Weißabgleich an einer deckenden Schicht von Marabu Maragloss GO Fluoresco 320 gelb herangezogen werden. Weitere Verbesserungen sind beispielsweise durch eine logische oder algebraische Verknüpfung der Weißabgleich-Parameter möglich.

Bei einer Variante der oben beschriebenen Prüfverfahren werden nach einem Weißabgleich der Kamera 31 an der Referenzoberfläche 72 die Weißabgleich-Parameter als Parameter des Betriebszustands der Kamera 31 ausgelesen bzw. erfasst. Anhand der Weißabgleich-Parameter werden das Beleuchtungsfilter 24 und das Beobachtungsfilter 13 identifiziert. Beleuchtungsfilter und Beobachtungsfilter können noch sicherer identifiziert werden, wenn eines der beiden Filter bereits bekannt ist oder wenn der Weißabgleich nacheinander an Referenzoberflächen oder Bereichen einer Referenzoberfläche mit unterschiedlichen optischen Eigenschaften durchgeführt wird.

Weißabgleich-Parameter sind - lineares Verhalten der Kamera vorausgesetzt - von der absoluten Helligkeit unabhängig, da sie nur Verhältnisse zwischen Signalen in den einzelnen Farbkanälen beschreiben. Somit sind die bei einem Weißabgleich ermittelten Weißabgleich-Parameter auch vom Abstand zwischen dem distalen Ende 12 der bildgebenden Einrichtung 10 und der Referenzoberfläche 72 unabhängig. Damit entfällt auch bei dieser Variante des Prüfungsverfahrens die Notwendigkeit einer genauen Positionierung des distalen Endes 12 des Endoskops bzw. der bildgebenden Einrichtung relativ zur Referenzoberfläche 72.

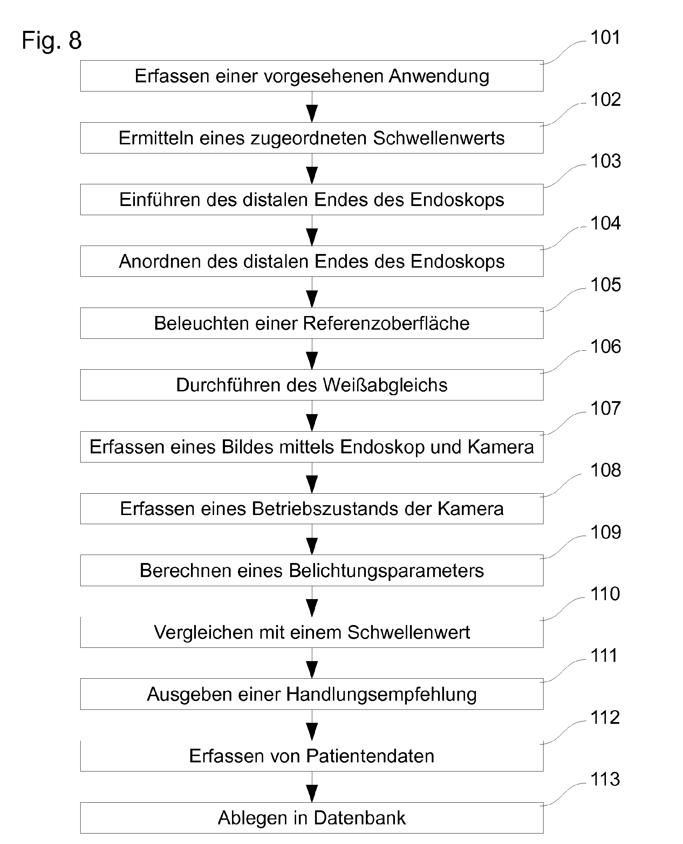

Figur 8 zeigt ein schematisches Flussdiagramm eines Verfahrens zum Prüfen eines optischen Untersuchungssystems mit einer bildgebenden Einrichtung, einer Kamera und einer Lichtquelle zur optischen Untersuchung eines Objekts. Obwohl das Verfahren auch an optischen Untersuchungssystemen und Prüfvorrichtungen anwendbar ist, die sich von den oben anhand der Figuren 1 und 2 dargestellten unterscheiden, werden nachfolgend zur Vereinfachung des Verständnisses beispielhaft Bezugszeichen aus den Figuren 1 und 2 verwendet. Das Verfahren kann Merkmale der oben beschriebenen Prüfverfahren und seiner beschriebenen Varianten aufweisen. Insbesondere kann das Verfahren eine Kombination mehrerer beschriebener Varianten sein.

Bei einem optionalen ersten Schritt 101 wird eine vorgesehene Anwendung des optischen Untersuchungssystems erfasst, beispielsweise an einer Benutzerschnittstelle nach einer entsprechenden Aufforderung. Bei einem optionalen zweiten Schritt 102 wird eine der vorgesehenen Anwendung zugeordnete Bedingung an einen Betriebszustand der Kamera 31 des optischen Untersuchungssystems ermittelt, beispielsweise durch Auslesen einer Look-up-Tabelle. Eine oder mehrere Bedingungen für den Betriebszustand der Kamera 31 können alternativ unveränderlich vorgegeben sein.

Bei einem dritten Schritt 103 wird ein distales Ende 12 einer bildgebenden Einrichtung 10, insbesondere eines Endoskops, durch eine Öffnung 43 in einen Hohlraum 42 in einem lichtdichten Gehäuse 41 eingeführt. Bei einem optionalen vierten Schritt 104, der unmittelbar nach dem dritten Schritt 103 oder gleichzeitig mit diesem ausgeführt werden kann, wird das distale Ende 12 der bildgebenden Einrichtung 10 in einer vorbestimmten Position und Richtung relativ zu einer in dem Hohlraum 42 angeordneten Referenzoberfläche 72 angeordnet. Dies erfolgt beispielsweise unterstützt durch eine Positionierungseinrichtung 50, die die bildgebende Einrichtung 10, insbesondere deren distales Ende 12 führt und/oder form- oder kraftschlüssig hält. Wie bereits oben erwähnt, kann der vierte Schritt 104 beispielsweise entfallen, wenn nachfolgend Quotienten aus Belichtungsparametern oder Akkumulatorwerten gebildet oder Weißabgleich-Parameter betrachtet werden, da hierbei der Einfluss des Abstands und der genauen Anordnung des distalen Endes12 der bildgebenden Einrichtung 10 relativ zur Referenzoberfläche näherungsweise eliminiert wird.

Bei einem fünften Schritt 105 wird die Referenzoberfläche 72 mit Beleuchtungslicht mit einem Beleuchtungsspektrum beleuchtet. Wenn die bildgebende Einrichtung ein Endoskop 10 ist, erfolgt die Beleuchtung insbesondere mittels des Endoskops bzw. über einen Beleuchtungs-Strahlengang im Endoskop 10. Bei einem optionalen sechsten Schritt 106 wird ein Weißabgleich, wie er oben beschrieben ist, durchgeführt während die Referenzoberfläche beleuchtet wird. Dabei werden beispielsweise Weißabgleich-Parameter WBGr, WBGb eingestellt.

Bei einem siebten Schritt 107 wird während des Beleuchtens der Referenzoberfläche 72 mittels der bildgebenden Einrichtung 10 durch eine Kamera 31 ein Bild erfasst. Bei einem achten Schritt 108 wird der während des siebten Schritts 107 vorliegende Betriebszustand der Kamera 31 erfasst, insbesondere aus der Kamera 31 oder der Kamerasteuerung 35 ausgelesen. Alternativ wird beispielsweise ein Rauschpegel oder ein Signal-Rausch-Abstand im erfassten Bild bestimmt, aus dem auf den Betriebszustand der Kamera 31 geschlossen wird. Der Betriebszustand der Kamera umfasst beispielsweise Weißabgleich-Parameter WBGr, WBGb, eine für alle Farbkanäle geltende Belichtungszeit, eine für alle Farbkanäle geltende Verstärkung, für einzelne Farbkanäle geltende Belichtungszeiten und Verstärkungen oder für einzelne Farbkanäle geltende Akkumulatorwerte.

Bei einem optionalen neunten Schritt 109 wird aus dem erfassten Betriebszustand der Kamera ein Belichtungsparameter ermittelt, insbesondere berechnet. Bei einem zehnten Schritt 110 wird der ermittelte Belichtungsparameter oder eine algebraische oder logische Verknüpfung von Belichtungsparametern oder von Akkumulatorwerten mit einem oder mehreren einer vorgesehenen Anwendung zugeordneten oder pauschal geltenden Schwellenwerten verglichen. Alternativ oder zusätzlich wird der Betriebszustand der Kamera bzw. die ihn charakterisierenden Parameter mit anderen der vorbestimmten Anwendung des optischen Untersuchungssystems zugeordneten und pauschal geltenden Bedingungen verglichen. Das Ergebnis des Vergleichs zeigt die Funktionsfähigkeit oder eine andere Eigenschaft des optischen Untersuchungssystems an.

Bei einem optionalen elften Schritt 111 wird eine Meldung ausgegeben, die eine Aussage über die Funktionsfähigkeit des optischen Untersuchungssystems, eine Handlungsempfehlung und/oder eine Handlungsanweisung umfassen kann. Bei einem zwölften Schritt 112, der auch zu einem beliebigen anderen Zeitpunkt in dem Verfahren ausgeführt werden kann, werden Patientendaten erfasst, beispielsweise mittels einer Benutzerschnittstelle. Bei einem optionalen dreizehnten Schritt 113 werden die Patientendaten, das Ergebnis des Prüfverfahrens hinsichtlich der Funktionsfähigkeit oder hinsichtlich der anderen Eigenschaft des optischen Untersuchungssystems und optional das Ergebnis einer nachfolgenden oder vorangehenden Untersuchung eines Patienten mittels des optischen Untersuchungssystems in einer Datenbank abgelegt.

Ferner können Modellbezeichnungen, Seriennummern, Software- oder Firmware-Versionen und andere Daten von Komponenten des optischen Untersuchungssystems über eine Kommunikationsleitung 39 abgefragt und zur Dokumentation bzw. Protokollierung in der Datenbank abgelegt werden. Ferner kann in der Datenbank oder separat auf einem anderen Datenträger die Untersuchung des Patienten dokumentiert bzw. protokolliert werden. Dabei werden beispielsweise Bilder bzw. ein Videodatenstrom der Kamera 31 in der Datenbank (beispielsweise in einem MPEG-Format) oder auf einem Videoband abgelegt.

### Bezugszeichen

- 10: Endoskop
- 11: proximales Ende des Endoskops 10
- 12: distales Ende des Endoskops 10
- 13: Beobachtungsfilter des Endoskops 10
- 14: Okular am proximalen Ende des Endoskops 10
- 15: Kupplung am Endoskop 10 für Lichtleitkabel 19
- 17: Schaft des Endoskops 10
- 19: Lichtleitkabel zu Kopplung des Endoskops 10 mit der Lichtquellenvorrichtung 20
- 20: Lichtquellenvorrichtung
- 22: Lichtquelle der Lichtquellenvorrichtung 20
- 23: erste Sammellinse
- 24: Beleuchtungsfilter der Lichtquellenvorrichtung 20
- 25: zweite Sammellinse
- 26: Kupplung an der Lichtquellenvorrichtung 20 für Lichtleitkabel 19

- 31: Kamera
- 33: Signalkabel
- 35: Kamerasteuerung
- 37: Bildschirm
- 39: Kommunikationsleitung
- 40: Prüfvorrichtung
- 41: lichtdichtes Gehäuse der Prüfvorrichtung 40
- 42: Hohlraum im lichtdichten Gehäuse 41
- 43: Öffnung im lichtdichten Gehäuse 41

- 50: erste Positionierungseinrichtung

- 70: Referenzkörper der Prüfvorrichtung 40
- 72: Referenzoberfläche am Referenzkörper 70
- 75: Indikatorbereich an der Referenzfläche 72
- 76: Referenzbereich an der Referenzfläche 72

- 81: Fluoreszenz-Anregungsspektrum
- 82: Fluoreszenz-Abregungsspektrum
- 83: Transmissionsspektrum des Beleuchtungsfilters
- 83L: Transmissionsspektrum des Beleuchtungsfilters für PDD
- 83F: Transmissionsspektrum des Beleuchtungsfilters für AF
- 84: Transmissionsspektrum des Beobachtungsfilters
- 84L: Transmissionsspektrum des Beobachtungsfilters für PDD
- 84F: Transmissionsspektrum des Beobachtungsfilters für AF
- 85: Produkt aus 83L und 84F
- 86: Produkt aus 83F und 84L
- 87: Produkt aus 83L und 84L
- 88: Produkt aus 83F und 84F

- 101: erster Schritt (Erfassen einer vorgesehenen Anwendung)
- 102: zweiter Schritt (Ermitteln eines Schwellenwerts)
- 103: dritter Schritt (Einführen des distalen Endes des Endoskops)
- 104: vierter Schritt (Anordnen des distalen Endes des Endoskops)
- 105: fünfter Schritt (Beleuchten einer Referenzoberfläche)
- 106: sechster Schritt (Durchführen eines Weißabgleichs)
- 107: siebter Schritt (Erfassen eines Bilds mittels Endoskop und Kamera)
- 108: achter Schritt (Erfassen eines Betriebszustands der Kamera)
- 109: neunter Schritt (Berechnen eines Belichtungsparameters)
- 110: zehnter Schritt (Vergleichen mit einem Schwellenwert)
- 111: elfter Schritt (Ausgeben einer Handlungsempfehlung)
- 112: zwölfter Schritt (Erfassen von Patientendaten)
- 113: dreizehnter Schritt (Ablegen in Datenbank)

## Patentansprüche

1. Verfahren zum Prüfen eines optischen Untersuchungssystems, nämlich eines Exo- oder Endoskopiesystems, insbesondere für die photodynamische Diagnostik, mit einer bildgebenden Einrichtung (10), einer Kamera (31) und einer Lichtquelle (22) zur optischen Untersuchung eines Objekts, mit folgenden Schritten:
Beleuchten (105) einer Referenzoberfläche (72) mit vorbestimmten optischen Eigenschaften mit Beleuchtungslicht der Lichtquelle (22);
Erfassen (107) eines Bilds der Referenzoberfläche (72) mittels der bildgebenden Einrichtung (10) und der Kamera (31);
Erfassen (108) eines Betriebszustands der Kamera (31), der während des Erfassens (107) des Bilds vorliegt;
Bestimmen (110) der Funktionsfähigkeit oder einer anderen Eigenschaft des Untersuchungssystems anhand des erfassten Betriebszustands,
wobei der Schritt des Beleuchtens (105) nacheinander ein Beleuchten der Referenzoberfläche (72) mit Beleuchtungslicht mit einem ersten Spektrum (81L) und
ein Beleuchten der Referenzoberfläche (72) mit Beleuchtungslicht mit einem zweiten Spektrum (81F) umfasst,
ein erster Betriebszustand der Kamera (31), der während des Erfassens (107) eines Bilds bei Beleuchten (105) der Referenzoberfläche (72) mit dem Beleuchtungslicht mit dem ersten Spektrum (81L) vorliegt, erfasst wird,
ein zweiter Betriebszustand der Kamera, der während des Erfassens (107) eines Bilds bei Beleuchten (105) der Referenzoberfläche (72) mit dem Beleuchtungslicht mit dem zweiten Spektrum (81F) vorliegt, erfasst wird,
und die Funktionsfähigkeit oder die andere Eigenschaft anhand des ersten Betriebzustands und des zweiten Betriebszustands bestimmt wird.

2. Verfahren nach dem vorangehenden Anspruch, ferner mit folgendem Schritt:
Anordnen (104) des distalen Endes (12) der bildgebenden Einrichtung (10) an einer vorbestimmten Position relativ zu der Referenzoberfläche (72).

3. Verfahren nach einem der vorangehenden Ansprüche, ferner mit folgenden Schritten:
Erfassen (101) einer vorgesehenen Anwendung des optischen Untersuchungssystems;
Ermitteln (102) einer Bedingung, die der vorgesehenen Anwendung des optischen Untersuchungssystems zugeordnet ist, wobei die Funktionsfähigkeit für die vorgesehene Anwendung oder eine andere vorbestimmte Eigenschaft des optischen Untersuchungssystems vorliegt, wenn der Betriebszustand der Bedingung entspricht.

4. Verfahren nach einem der vorangehenden Ansprüche, bei dem die Referenzoberfläche (72) mit einer Bestrahlungsstärke beleuchtet wird, die einen vorbestimmten Bruchteil der bei der vorgesehenen Verwendung des optischen Untersuchungssystems angewandten Bestrahlungsstärke beträgt.

5. Verfahren nach einem der vorangehenden Ansprüche, bei dem:
der Schritt des Erfassens (108) des Betriebszustands ein Ermitteln eines Belichtungsparameters umfasst;
der Schritt des Bestimmens der Funktionsfähigkeit oder einer anderen Eigenschaft ein Vergleichen (110) des ermittelten Belichtungsparameters mit einem vorbestimmt Schwellenwert umfasst.

6. Verfahren nach einem der vorangehenden Ansprüche, bei dem der Schritt des Bestimmens der Funktionsfähigkeit oder einer anderen Eigenschaft folgende Schritt umfasst:
Ermitteln eines Indikatorparameters aus dem erfassten ersten Betriebszustand und dem erfassten zweiten Betriebszustand;
Vergleichen des ermittelten Indikatorparameters mit einem Schwellenwert.

7. Verfahren nach einem der vorangehenden Ansprüche, bei dem:
das Erfassen (108) eines Betriebszustands ein Ermitteln je eines Belichtungsparameters für jeden einer Mehrzahl von Farbkanälen umfasst.

8. Verfahren nach einem der vorangehenden Ansprüche, bei dem:
das Erfassen (108) eines Betriebszustands ein Erfassen eines Weißabgleich-Parameters umfasst.

9. Verfahren nach einem der vorangehenden Ansprüche, bei dem:
das Erfassen (108) eines Betriebszustands ein Erfassen eines Rauschpegels oder eines Signal-Rausch-Abstands in dem erfassten Bild umfasst.

10. Verfahren nach einem der vorangehenden Ansprüche, ferner mit folgenden Schritten:
Erfassen (112) von Patientendaten;
Ablegen (113) einer Information über die Funktionsfähigkeit oder die andere Eigenschaft sowie der Patientendaten in einer Datenbank.

11. Computer-Programm mit Programmcode zur Durchführung oder Steuerung eines Verfahrens nach einem der vorangehenden Ansprüche, wenn das Computer-Programm auf einem Computer oder einem Prozessor abläuft.

12. Steuerung (35) für ein optisches Untersuchungssystems mit einer bildgebenden Einrichtung (10), einer Kamera (31) und einer Lichtquelle (22) zur optischen Untersuchung eines Objekts, wobei die Steuerung (35) ausgebildet ist, um ein Verfahren nach einem der vorangehenden Verfahrensansprüche zu steuern.

## Claims

1. Method for testing an optical examination system, namely an exoscopy system or endoscopy system, in particular for photodynamic diagnostics, comprising an imaging apparatus (10), a camera (31) and a light source (22) for optically examining an object, comprising the following steps:
illuminating (105) a reference surface (72) with predetermined optical properties using illumination light from the light source (22);
acquiring (107) an image of the reference surface (72) by means of the imaging apparatus (10) and the camera (31);
acquiring (108) an operating state of the camera (31), which operating state is present during the acquisition (107) of the image;
determining (110) the functionality or another property of the examination system on the basis of the acquired operating state,
wherein the step of illuminating (105) comprises, in succession, an illumination of the reference surface (72) with illumination light having a first spectrum (81L) and an illumination of the reference surface (72) with illumination light having a second spectrum (81F),
a first operating state of the camera (31), which is present during the acquisition (107) of an image when illuminating (105) the reference surface (72) with the illumination light having the first spectrum (81L), is acquired,
a second operating state of the camera, which is present during the acquisition (107) of an image when illuminating (105) the reference surface (72) with the illumination light having the second spectrum (81F), is acquired,
and the functionality or the other property is determined on the basis of the first operating state and the second operating state.

2. Method according to the preceding claim, furthermore comprising the following step:
arranging (104) the distal end (12) of the imaging apparatus (10) at a predetermined position relative to the reference surface (72).

3. Method according to one of the preceding claims, furthermore comprising the following steps:
acquiring (101) an intended application of the optical examination system;
establishing (102) a condition which is associated with the intended application of the optical examination system, wherein the functionality for the intended application or another predetermined property of the optical examination system is present when the operating state corresponds to the condition.

4. Method according to one of the preceding claims, in which the reference surface (72) is illuminated with an irradiance which is a predetermined fraction of the irradiance applied during the intended use of the optical examination system.

5. Method according to one of the preceding claims, in which:
the step of acquiring (108) the operating state comprises an establishment of an exposure parameter;
the step of determining the functionality or another property comprises a comparison (110) of the established exposure parameter with a predetermined threshold.

6. Method according to one of the preceding claims, in which the step of determining the functionality or another property comprises the following steps:
establishing an indicator parameter from the acquired first operating state and the acquired second operating state;
comparing the established indicator parameter with a threshold.

7. Method according to one of the preceding claims, in which:
the acquisition (108) of an operating state comprises an establishment of respectively one exposure parameter for each of a plurality of colour channels.

8. Method according to one of the preceding claims, in which:
the acquisition (108) of an operating state comprises an acquisition of a white balance parameter.

9. Method according to one of the preceding claims, in which:
the acquisition (108) of an operating state comprises an acquisition of a noise level or of a signal-to-noise ratio in the acquired image.

10. Method according to one of the preceding claims, furthermore comprising the following steps:
acquiring (112) patient data;
storing (113) information about the functionality or the other property, and also the patient data, in a database.

11. Computer program comprising program code for performing or controlling a method according to one of the preceding claims, if the computer program is executed on a computer or a processor.

12. Controller (35) for an optical examination system comprising an imaging apparatus (10), a camera (31) and a light source (22) for optically examining an object, wherein the controller (35) is embodied to control a method according to one of the preceding method claims.

## Revendications

1. Procédé destiné à tester un système d'examen optique, à savoir un système d'endoscopie ou d'exoscopie, en particulier pour le diagnostic photodynamique, comportant un dispositif d'imagerie (10), une caméra (31) et une source de lumière (22) pour examiner optiquement un objet, comprenant les étapes consistant à :
éclairer (105) une surface de référence (72) ayant des propriétés optiques prédéterminées avec la lumière d'éclairement de la source de lumière (22) ;
détecter (107) une image de la surface de référence (72) au moyen du dispositif d'imagerie (10) et de la caméra (31) ;
détecter (108) un état de fonctionnement de l'appareil (31), qui est présent lors de la détection (107) de l'image ;
déterminer (110) l'aptitude au fonctionnement ou une autre propriété du système d'examen sur la base de l'état de fonctionnement détecté,
dans lequel l'étape d'éclairement (105) consiste à éclairer séquentiellement la surface de référence (72) avec une lumière d'éclairement ayant un premier spectre (81L) et à éclairer la surface de référence (72) avec une lumière d'éclairement ayant un second spectre (81F),
un premier état de fonctionnement de la caméra (31), qui est présent pendant la détection (107) d'une image lors de l'éclairement (105) de la surface de référence (72) avec la lumière d'éclairement ayant le premier spectre (81L), est détecté,
un second état de fonctionnement de la caméra, qui est présent pendant la détection (107) d'une image lors de l'éclairement (105) de la surface de référence (72) avec la lumière d'éclairement ayant le second spectre (81F), est détecté,
et l'aptitude au fonctionnement ou l'autre propriété est déterminée sur la base du premier état de fonctionnement et du second état de fonctionnement.

2. Procédé selon la revendication précédente, comprenant en outre l'étape consistant à :
disposer (104) l'extrémité distale (12) du dispositif d'imagerie (10) à une position prédéterminée par rapport à la surface de référence (72).

3. Procédé selon l'une quelconque des revendications précédentes, comprenant en outre les étapes consistant à :
détecter (101) une application envisagée du système d'examen optique ;
déterminer (102) une condition associée à l'application envisagée du système d'examen optique, dans lequel l'aptitude au fonctionnement pour l'application envisagée ou une autre propriété prédéterminée du système d'examen optique est présente si l'état de fonctionnement correspond à la condition.

4. Procédé selon l'une quelconque des revendications précédentes, dans lequel la surface de référence (72) est éclairée avec une intensité de rayonnement qui est une fraction prédéterminée de l'intensité de rayonnement appliquée lors de l'utilisation prévue du système d'examen optique.

5. Procédé selon l'une quelconque des revendications précédentes, dans lequel :
l'étape de détection (108) de l'état de fonctionnement consiste à déterminer un paramètre d'exposition ;
l'étape de détermination de l'aptitude au fonctionnement ou d'une autre propriété consiste à comparer le paramètre d'exposition déterminé à une valeur de seuil prédéterminée (110).

6. Procédé selon l'une quelconque des revendications précédentes, dans lequel l'étape de détermination de l'aptitude au fonctionnement ou d'une autre propriété comprend l'étape consistant à :
déterminer un paramètre indicateur à partir du premier état de fonctionnement détecté et du second état de fonctionnement détecté ;
comparer le paramètre indicateur déterminé à une valeur de seuil.

7. Procédé selon l'une quelconque des revendications précédentes, dans lequel :
la détection (108) d'un état de fonctionnement consiste à déterminer chaque paramètre d'exposition pour chacun d'une pluralité de canaux de couleurs.

8. Procédé selon l'une quelconque des revendications précédentes, dans lequel :
la détection (108) d'un état de fonctionnement consiste à détecter un paramètre de balance des blancs.

9. Procédé selon l'une quelconque des revendications précédentes, dans lequel :
la détection (108) d'un état de fonctionnement consiste à détecter un niveau de bruit ou un rapport signal à bruit dans l'image détectée.

10. Procédé selon l'une quelconque des revendications précédentes, comprenant en outre les étapes consistant à :
détecter (112) des données des patients ;
stocker (113) une information concernant l'aptitude au fonctionnement ou l'autre propriété et les données des patients dans une base de données.

11. Programme d'ordinateur comportant un code de programme destiné à mettre en oeuvre ou commander un procédé selon l'une quelconque des revendications précédentes lorsque le programme d'ordinateur est exécuté sur un ordinateur ou un processeur.

12. Dispositif de commande (35) destiné à un système d'examen optique comportant un dispositif d'imagerie (10), une caméra (31) et une source de lumière (22) pour examiner optiquement un objet, dans lequel le dispositif de commande (35) est conçu pour commander un procédé selon l'une quelconque des revendications de procédé précédentes.
